# EUROPEAN PATENT APPLICATION

(11) **EP 1 867 973 A1**
(43) Date of publication of application: **19.12.2007**
(21) Application number: 06731319.7
(22) Date of filing: 06.04.2006
(51) Int. Cl.: G01N 1/10, C12Q 1/04, C12Q 1/37, G01N 33/48

(54) **SAMPLING DEVICE FOR VISCOUS SAMPLE, HOMOGENIZATION METHOD FOR SPATUM AND METHOD OF DETECTING MICROBE**

(30) Priority: 08.04.2005 JP 2005112297; 08.04.2005 JP 2005112298; 02.05.2005 JP 2005134220; 26.09.2005 JP 2005277167
(71) Applicant: Eisai R&D Management Co., Ltd., Tokyo 112-8088 (JP)
(72) Inventor: USUI, Mitsugu, Yokohama-shi, Kanagawa 2260027 (JP); MORISHITA, Takeharu No.507, Kikuna Residencia, Yokohama-shi, Kanagawa 22200 (JP)
(74) Representative: Grünecker, Kinkeldey, Stockmair & Schwanhäusser Anwaltssozietät
(86) International application number: PCT/JP2006/307371
(87) International publication number: WO 2006/109693

(57) **Abstract**

It is intended to provide: a sampling device for quantitatively sampling a liquid sample having a high viscosity such as sputum; an auxiliary jig therefor; an agent for homogenizing sputum by which sputum can be quickly treated under mild conditions and a microbe contained in the sputum can be detected at an improved stability; a homogenization device; a homogenization method; a method of detecting a microbe by using the above method; a separation device for conveniently, quickly and efficiently collecting a microbe contained in sputum; a method of collecting a microbe; and a method of detecting a microbe by using the above method. A sampling device for a viscous sample includes: a tubular body; a gasket; a long rod-like plunger; a plate-shaped nozzle that has substantially the same diameter as that of a straight section of the tubular body and is adapted to close a distal end of the tubular body; and a slit formed in the plate-like nozzle, the slit having a predetermined shape adapted to serve as an aspiration and discharge port for aspirating and discharging the viscous sample.

SEQUENCE LISTING

<110>  EISAI R&D MANAGEMENT CO., LTD.

<120>  Sampling Device for viscous sample, homogenization
       method for sputum and method of detecting microbe

<130>  EP54167FZpau

<140>  EP 06 731 319.7
<141>  2006-04-06

<150>  PCT/JP2006/307371
<151>  2006-04-06

<150>  JP 2005-112297
<151>  2005-04-08

<150>  JP 2005-112298
<151>  2005-04-08

<150>  JP 2005-134220
<151>  2005-05-02

<150>  JP 2005-277167
<151>  2005-09-26

<160>  4

<170>  PatentIn version 3.1

<210>  1
<211>  42
<212>  DNA
<213>  Artificial

<220>
<223>  Description of Artificial Sequence; Synthetic

<220>
<221>  misc_feature
<222>  (42)..(42)
<223>  Aminolink attached at the 3'end


<400>  1
cgtctttcac ttttgaacca tgcggttcaa aatattatcc gg                    42


<210>  2
<211>  81
<212>  DNA
<213>  Artificial

<220>
<223>  Description of Artificial Sequence; Synthetic

<400>  2
catgtctcgt gtcttgcatc ctgctacagt gaacaccatc gttctcgaca tagaccagtc      60

atctataagt gacagcaaga c                                                81


<210>  3
<211>  60
<212>  DNA
<213>  Artificial

<220>

<223>  Description of Artificial Sequence; Synthetic

<400>  3
catgtctcgt gtcttgcatc ctgctacagt gaacaccatc gttctcgaca tagaccagtc      60


<210>  4
<211>  60
<212>  DNA
<213>  Artificial

<220>
<223>  Description of Artificial Sequence; Synthetic

<220>
<221>  misc_feature
<222>  (1)..(1)
<223>  digoxigenin label attached at the 5'end


<400>  4
gatgcaagac acgagacatg gatggtgttc actgtagcag gactggtcta tgtcgagaac      60

## Description

### Technical Field

The present invention relates to: a sampling device for quantitatively sampling a liquid sample having a high viscosity such as sputum; an auxiliary jig therefor; an agent for homogenizing sputum by which sputum can be quickly treated under mild conditions and microbe contained in the sputum can be detected at an improved stability; a homogenization device; a homogenization method; a method of detecting a microbe by using the above method; a separation device for conveniently, quickly and efficiently collecting a microbe contained in sputum; a method of collecting a microbe; and a method of detecting a microbe by using the above method.

### Background Art

It has heretofore been a common practice to use a dropper, a pipette or a syringe as a sampling device when a liquid sample which is a subject of a cultivation test (identification of microbes) or the like is collected and sampled. However, such devices have had a problem that while a liquid sample having a relatively low viscosity can be sampled more or less quantitatively, a liquid sample having a relatively high viscosity such as sputum (hereinafter, referred to as a "viscous sample") is sampled with considerably deteriorated quantitativeness.

That is, with a conventional dropper or a pipette, which aspirates or discharges a viscous sample via air pressure, an entire amount of the aspirated viscous sample can not be discharged since a part of the viscous sample gets stuck on an inner wall of the dropper or pipette particularly when the viscous sample is discharged therethrough.

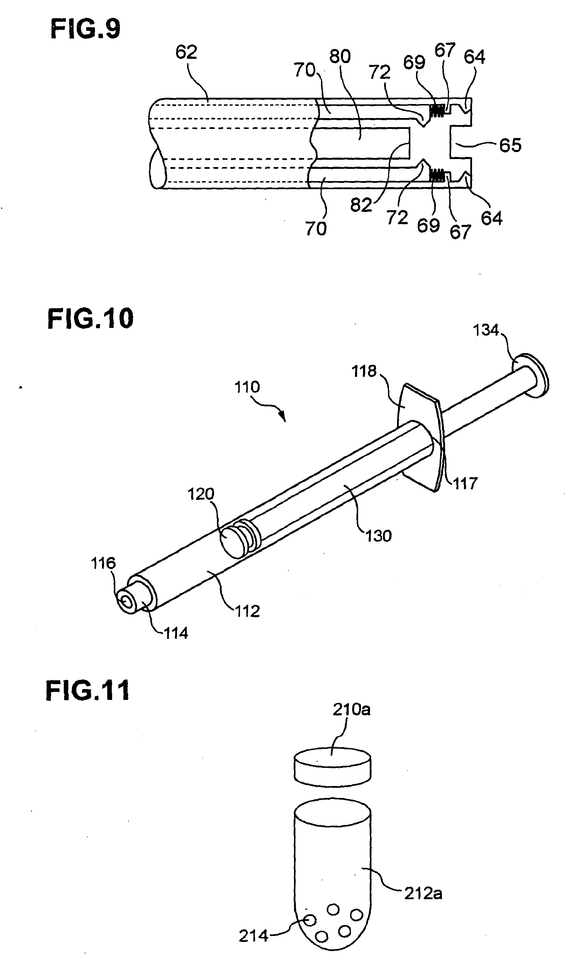

Further, as shown in Fig. 10, a conventional syringe 110 includes a tubular body 112 having a long, straight part that is hollow and has a small-diameter nozzle 114 protruding therefrom on a side of a leading edge, the nozzle 114 being provided with a circular aspiration and discharge port 116 at a tip of the nozzle 114, and an aperture 117 on a base side of the body section having a flange 118 on a periphery of the aperture 117. The syringe 110 also includes a gasket 120 slidably inserted in a longitudinal direction along and in contact with an inner wall of the cylinder 112 and a long, rod-like plunger 130 having a leading edge engaged with the gasket 120 and a base edge serving as an operation head 134 in order to slidably operate the gasket 120 (see, Fig. 10 and Patent Documents 1 and 2, etc.).

In the case of the syringe 110 as such, while the viscous sample does not remain on the inner wall of the tubular body 112 owing to the gasket 120, the protruded nozzle 114 causes the viscous sample to remain in the inside of the nozzle 114, so in this case too the total amount of the aspirated viscous sample could not be discharged as was expected. In addition, in the case of the conventional syringe 110, a relatively large aspiration power and discharge power are required, so a heavy burden rests on the operator. As other factors, mention may be made of problems that with the conventional syringe 110, the viscous sample sticks and does not separate in a clear cut manner upon aspiration and discharge, so it is difficult to collect a necessary amount of the viscous sample and that the sticky viscous sample form strings, which attach to other sites, thus causing an issue of environmental public health.

On the other hand, when specified ingredients contained in sputum, in particular pathogenic microbes in sputum of patients suffering from respiratory infection are to be detected and determined, the sputum of a patient is collected and incubated to detect a pathogenic microbe. However, the sputum of a patient generally has a high viscosity and it is difficult to have the sputum specimen uniformly smeared on a medium, resulting in an error in tests results. Also, in gene tests, it is difficult to determine an exact number of microbes in the sputum as they are. Therefore, it is necessary to pretreat sputum to make the ingredients contained in the sputum uniform, so detection and determination of a specified ingredient can be performed with ease.

As a measure for such, a lot of pretreatment methods have been considered. As a pretreatment method in the detection of an acid-fast bacterium or tubercule bacillus, for example, Non-patent Document 1 recommends an NALC-NaOH method that involves use of N-acetyl-L-cysteine (NALC) which is a reducing substance, and the method is widely used (see, for example, Patent Document 3). However, the NALC-NaOH method has a problem that when the method is to be applied to detection of general bacteria such as Haemophilus influenzae, it requires severe treatment conditions, thus giving a large damage to the bacteria. Methods that require mild pretreatment conditions include a method that involves use of semi-alkaline protease as an enzyme. However, the method could not achieve a satisfactory homogenization treatment for a viscous sample having a high viscosity such as purulent sputum. Then, there is known a method that involves use of semi-alkaline protease and the NALC-NaOH method in combination (see, for example, Patent Document 4). However, this method is cumbersome in operation and takes a long time. Further, there is a problem that the viable cell number of a sample decreases depending on the required time of pretreatment of sputum, which may make it difficult to exactly determine the pathogenic bacteria.

Further, a method that involves use of a vortex mixer predominates as a suspension method in which the above-mentioned pretreatment liquid is used. Therefore, no sufficient homogenization treatment could be performed for samples having high viscosity strengths, such as purulent sputum, in some cases.
Accordingly, as shown in Patent Document 5, there has been proposed homogenization of sputum by using a sputum treating device having placed therein a spherical solid material. The document describes glass or quartz as the spherical solid material. However, it could take a long time for homogenizing purulent sputum by using the above-mentioned sputum treating device and it has been sometimes the case that sufficient homogenization treatment could not be performed with the device.

Further, samples treated with the above-mentioned pretreatment liquid contains various types of substances such as microbes, human-derived cells, dust and so on in admixture, so when such samples are used in testing genes and proteins and so on, false-positive judgment may be done in many cases. Therefore, for example, Patent Document 6 discloses a method of collecting microbes from a specimen by adsorbing microbes only with an adsorption carrier after pretreating the specimen. However, this method requires two operations, i.e., adsorption and peeling, so the method has problems that the operation is cumbersome and the yield is poor. A method is known in which a specimen is filtered through a filter to trap microbes thereon (Patent Document 7). However, it would be necessary to improve this method in yield and separation of human cells, blood cells, dust, and so on.

On the other hand, Patent Document 8 describes a method that involves pretreating a specimen and then filtering the specimen through a filter member having at least two types of filter membranes to obtain a sample for immunochromatography. This method is intended to avoid clogging of a membrane carrier for chromatography with a sample when immunochromatography is performed but not to collect microbes; the document discloses nothing on collecting microbes. Further, the document describes only glass fiber filter paper and glass filter as a filter membrane.
Patent Document 1: JP 05-3318 B
Patent Document 2: JP 01-21989 B
Patent Document 3: JP 2004-344108 A
Patent Document 4: JP 2002-65249 A
Patent Document 5: JP 01-291160 A
Patent Document 6: JP 2001-112497 A
Patent Document 7: JP 2004-180551 A
Patent Document 8: JP 2005-24325 A
Non-Patent Document 1: "New Guideline 2000 for Testing Tubercule Bacillus", published by Japan Anti-Tuberculosis Association, Foundation.

### Disclosure of the Invention

### Problems to be solved by the Invention

The present invention has been achieved in view of the above-mentioned problems. Firstly, it is an object of the present invention to provide a sampling device for viscous sample and an auxiliary jig therefor that improves quantitativeness when sampling a viscous sample such as sputum, reduces a burden to an operator with a relatively low aspiration power and discharge power, improves clear-cutting property upon aspiration and discharge of the viscous sample, and provides those with a less problem on environmental sanitation. Secondly, it is an object of the present invention to provide a homogenization agent, a homogenization device, a homogenization method, and a method of detecting a microbe each of which enables homogenization treatment of sputum, conveniently and quickly performs homogenization treatment of sputum under mild conditions, and enables detection of a microbe present in the sputum more stably. Thirdly, it is an object of the present invention to provide a separation device for conveniently, quickly and efficiently collecting a microbe contained in sputum, a method of collecting a microbe, and a method of detecting a microbe by using the above method.

### Means for Solving the Problems

The inventors of the present invention have made extensive studies with a view to achieving the above objects and as a result, they have found that: firstly, in sampling a viscous sample such as sputum, a change in shape of the tip of a syringe enables improvement of quantitativeness and operability, so the viscous sample can be easily and safely collected; secondly, in the method of pretreating sputum, use of cysteine protease which is one of a protease, in particular bromelain enables efficient pretreatment; thirdly, in the method of pretreating sputum, stirring a sample by using particles containing one or more members selected from the group consisting of metals and oxides thereof enables convenient and quick pretreatment in the case of a sample having a high viscosity such as purulent sputum; and fourthly, in detecting microbes from sputum, use of a separation device having three types of filters enables efficient separation of microbes from cells, blood cells and dust and so on. The present invention has been accomplished based on these findings.

To solve the problems, a sampling device for a viscous sample of the present invention includes: a tubular body (10) with a closed distal end and an open proximal end, having a long straight middle section that is hollow inside, and a flange on an outer periphery of the proximal end; a gasket (20) inserted in the tubular body so that the gasket is brought into contact with an inner wall of the tubular body and slidable in a longitudinal direction along the tubular body; and a long rod-like plunger (30) having a distal end serving as an engagement convex that engages with the gasket and a proximal end serving as an operation head adapted to slidably operate the gasket when the operation head is operated; a plate-like nozzle (14) that has substantially the same diameter as that of the straight middle section of the tubular body and is adapted to close the distal end of the tubular body; and a slit (16) formed in the plate-like nozzle, the slit having a predetermined shape adapted to serve as an aspiration and discharge port for aspirating and discharging the viscous sample.

The slit is preferably positioned near a central part of the plate-like nozzle (14). In addition, the slit is desirably of a shape having at least one corner.
The shape of the slit is in the form of preferably any one of a cross (X-shape), Y-shape and W-shape. The width of the slit is preferably 0.5 mm to 2.0 mm, more preferably 0.5 mm to 1.5 mm.

The viscous sample is not particularly limited as far as it is a liquid sample having a high viscosity that serves as a subject of an examination, analysis or test. The device of the present invention is particularly suitable when the viscous sample is sputum.

An auxiliary jig for the sampling device for a viscous sample of the present invention is an auxiliary jig (40, 60) for the above-mentioned sampling device for a viscous sample, in which the auxiliary jig is adapted to make the operation head of the sampling device operable.

The auxiliary jig (40) for the sampling device preferably includes: a long tubular external fitted tube member (42) including at a distal end thereof an engagement section engageable with the flange of the tubular body in the sampling device, a proximal end provided with an opening, and a guide groove in a straight middle section of the tube member (42); and an internal fitted shaft member (50) slidably and coaxially arranged inside the external fitted tube member, and including an engagement section that is engageable with the operation head of the plunger in the sampling device at a distal end thereof, and an operation lever along the guide groove, and a press head at a proximal end thereof.

The auxiliary jig (60) for a sampling device is an auxiliary jig (60) for the above-mentioned sampling device preferably including: a long tubular external fitted tube member (62) including on an inner periphery of a distal end thereof a fitting convex fittable with the flange of the tubular body in the sampling device and a proximal end thereof provided with an opening; an intermediate fitted tube member (70) slidably and coaxially arranged inside the external fitted tube member and including a fitting convex provided on an inner periphery of a distal end thereof adapted to be fittable with the operation head of the plunger in the sampling device and a biasing unit and an engagement unit with respect to the external fitted tube member; and an internal fitted shaft member (80) slidably and coaxially arranged inside the intermediate fitted tube member and including a distal end serving as an abutting end that abuts the operation head of the plunger in the sampling device and a proximal end serving as a press head.

The homogenization agent for sputum according to the present invention includes cysteine protease. The cysteine protease is preferably cysteine protease of plant origin, more preferably one or more members selected from the group consisting of bromelain, papain, chymopapain, and ficin, and particularly preferably bromelain. Preferably, the homogenization agent of the present invention further contains sodium chloride and Tris buffer solution. The homogenization agent of the present invention is advantageously used in the pretreatment in the method of detecting a microbe.

The homogenization device for sputum according to the present invention houses particles containing one or more members selected from the group consisting of metals and oxides thereof. Preferably, the metals and oxides thereof include stainless steel, iron or alumina. It is preferable that the above metals and oxides thereof include stainless steel, iron and alumina. It is preferable that the particles be spherical particles having a diameter of 1 mm or more and 10 mm or less and more preferably spherical particles having a diameter of 4 mm or more and 10 mm or less.

In a first aspect, the homogenization method for sputum according to the present invention includes treating sputum with a homogenization agent of the present invention. The homogenization method of the present invention is particularly preferable as a pretreatment in detecting a microbe.

In a second aspect, the homogenization method for sputum according to the present invention includes stirring a sample containing subject sputum in the presence of one or more members selected from the group consisting of metals and oxides thereof.

Preferably, the metals and oxides thereof are stainless steel, iron and alumina. Note that in the present specification, "stainless steel" is also referred to as simply "stainless".
The particles are preferably spherical particles having a diameter of 1 mm or more and 10 mm or less, more preferably spherical particles having a diameter of 4 mm or more and 10 mm or less.

It is preferable that the sample contain a pretreating agent for sputum.
It is preferable that the pretreating agent for sputum contain a protease. It is preferable that the protease include one or more members selected from the group consisting of semi-alkaline protease and cysteine protease. Further, it is preferable that the cysteine protease be one or more members selected from the group consisting of bromelain, papain, chymopapain, and ficin. Also, it is preferable that the pretreating agent for sputum be the homogenizing agent of the present invention.

The homogenization method of the present invention is particularly advantageous as a pretreatment in the method of detecting a microbe.

A method of collecting a microbe from sputum of the present invention is a method of collecting a microbe from homogenized sputum, including: providing a separation device having at least three types of filters each allowing passage of a subject microbe and made of an organic material; and passing the homogenized sputum through the separation device to obtain a filtrate containing the subject microbe.

The separation device is provided with a first filter, a second filter, and a third filter in the order in which the homogenized sputum passes, and the filters have respective pore diameters preferably becoming smaller in series toward downstream. It is preferable that: the third filter have a pore diameter of 0.1 µm or more and 10 µm or less, more preferably 2 µm or more and 7 µm or less; the second filter have a pore diameter of 5 µm or more, more preferably 10 µm or more and 30 µm or less; and the first pore diameter have a pore diameter of 5 µm or more, more preferably 20 µm or more, still more preferably 80 µm or more and 120 µm or less.

The organic material is preferably at least one member selected from the group consisting of polycarbonate, polytetrafluoroethylene, polyamide, cellulose, and polyethylene.
The preferable subject microbes include such microbes as virus, Rickettsia, bacteria or fungi.
In the method of collecting the microbes according to the present invention, the homogenization method for sputum is not particularly limited and the homogenization method of the present invention is advantageously used.

The separation device for microbes according to the present invention is the above-mentioned separation device having at least three types of filters through which the subject microbes can pass and which are made from an organic material, and is used in the method of collecting microbes according to the present invention.

A first aspect of a method of detecting a microbe of the present invention includes: treating a subject sputum by the homogenization method for sputum of the present invention; and detecting a microbe present in the subject sputum.

A second aspect of a method of detecting a microbe of the present invention includes: detecting a microbe from a filtrate obtained by the method of collecting a microbe of the present invention.

In the method of detecting microbes according to the present invention, the microbes that are preferable include virus, Rickettsia, bacteria, and fungi.

### Effects of the Invention

According to the present invention, significant effects can be obtained in that there can be provided a sampling device for a viscous sample and an auxiliary jig therefor that can improve quantitativeness when sampling a viscous sample such as sputum, reduce a burden to an operator with a relatively low aspiration and discharge powers, improve clear-cutting property upon aspiration and discharge of the viscous sample, and give a less problem on environmental sanitation.

According to the present invention, sputum can be homogenized more conveniently and quickly and microbes can be detected more stably than by the conventional pretreatment of sputum. Further, according to the present invention, microbes contained in sputum can be collected more conveniently, quickly and efficiently than by the conventional method.

### Brief Description of the Drawings

Fig. 1 is a perspective view showing a sampling device for a viscous sample according to the present invention.
Fig. 2 is an exploded perspective view shown in Fig. 1.
Fig. 3 is a plan view showing various examples of the shape of a slit (aspiration and discharge port) of a plate-like nozzle in the sampling device of the present invention.
Fig. 4 is an enlarged explanatory diagram shown in Fig. 3(a).
Fig. 5 is a schematic explanatory diagram of the device of the present invention used in measuring aspiration power and discharge power, with (a) illustrating the case of aspiration and (b) illustrating the case of discharge powder.
Fig. 6 is an explanatory side view of an example of the auxiliary jig for the sampling device of the present invention.
Fig. 7 is an enlarged perspective view of a main part of the auxiliary jig shown in Fig. 6(a).
Fig. 8 is an explanatory side view of another example of the auxiliary jig for the sampling device of the present invention.
Fig. 9 is an enlarged view of a main part of the auxiliary jig shown in Fig. 8(a).
Fig. 10 is a perspective explanatory diagram of a conventional syringe.
Fig. 11 is a schematic explanatory diagram of a first example of a homogenization device for sputum according to the present invention.
Fig. 12 is a schematic explanatory diagram of a second example of the homogenization device for sputum according to the present invention.
Fig. 13 is a schematic explanatory diagram of a third example of the homogenization device for sputum according to the present invention.
Fig. 14 is a schematic explanatory diagram of a fourth example of the homogenization device for sputum according to the present invention.
Fig. 15 is a schematic explanatory diagram illustrating an example of the method of collecting a microbe according to the present invention.
Fig. 16 is a schematic explanatory diagram illustrating another example of the method of collecting a microbe according to the present invention.
Fig. 17 is a schematic explanatory diagram illustrating an example of a separation device a microbe according to the present invention.
Fig. 18 is a schematic explanatory diagram illustrating an example of a kit of collecting a microbe including the separation device of the present invention.
Fig. 19 is a graph showing results of Examples 16 and 17.
Fig. 20 is a micrograph of a homogenized liquid before the filtration in Example 18, with (a) and (b) indicating the results at magnifications of 100 and 1000 folds, respectively.
Fig. 21 is a micrograph of a homogenized liquid after the filtration in Example 18, with (a) and (b) indicating the results at magnifications of 100 and 1000 folds, respectively.

### Description of Reference Numerals

2: a sampling device of the present invention, 10: a tubular body, 12: a straight middle section, 13: a closed part, 14, 14a~14j: plate-like nozzles, 15: a corner, 16, 16a~16j: slits, 17: an opening, 18: a flange, 20: a gasket, 22: a sealing rim, 24: a core, 30: a plunger, 32: an engagement protrusion, 34: an operation head, 40: an auxiliary jig for a sampling device of the present invention (one example), 42: an external fitted tube member, 43: an opening, 44: an engagement section, 45: a notch, 46: a tapered section, 48: a guide groove, 50: an internal fitted shaft member, 52: an engagement section, 53: a recess, 56: a press head, 58: an operation lever, 60: an auxiliary jig for a sampling device of the present invention (another example), 62: an external fitted tube member, 63: an opening, 64: a fitting convex, 65: a notch recess, 66: a tapered section, 67: a locking convex, 68: a button hole, 69: a biasing unit (spring), 70: an intermediate fitted tube member, 72: a fitting convex, 74: a locking unit (button), 76: a spring, 80: an internal fitted shaft member, 82: an abutting end, 84: a press head, 90: a weight holder, 92: a weight, 94: a sample container, 110: a syringe, 112: a tubular body, 114: a nozzle, 116: an aspiration and discharge port, 117: an opening, 118: a flange, 120: a gasket, 130: a plunger, 134: an operation head, P: a viscous sample, 210a~210d: lids of containers, 212a~212d: containers, 214: a metal particle, 216: a bottom of a container, 218: a bottom lid, 310a, 310b, 310c: separation devices for a microbe of the present invention, 311: a first filter, 312: a second filter, 313: a third filter, 314: a separation member, 315a, 315b, 315c: collecting containers, 316a, 316b, 316c: filter holders, 317a: a body of a filter holder, 318a, 318b: lid, 320: subject microbes, 322: a foreign substance, 324: homogenate, 330: filtrate, 332: supernatant, 334: precipitation, 340: a syringe.

### Best Mode for Carrying Out the Invention

Hereinafter, embodiments of the present invention will be described referring to the attached drawings. However, the illustrated examples are merely exemplary and it is needless to say that various modifications may be made without departing from the technical concept of the present invention.

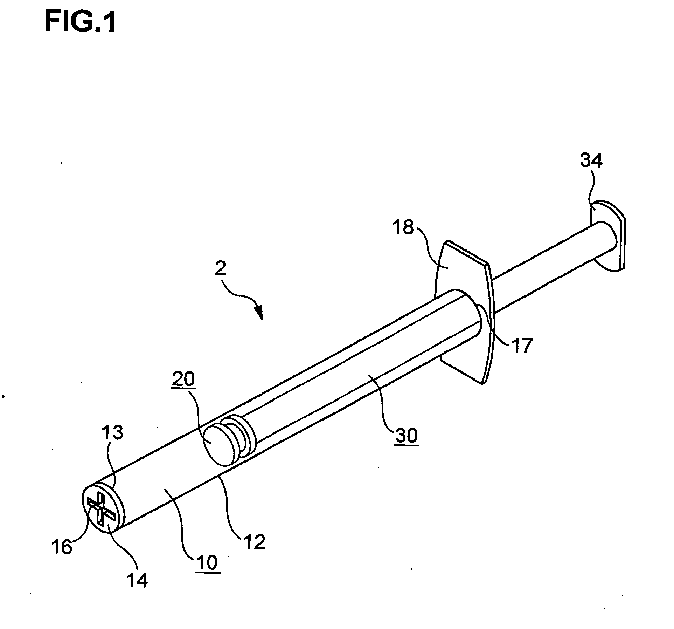

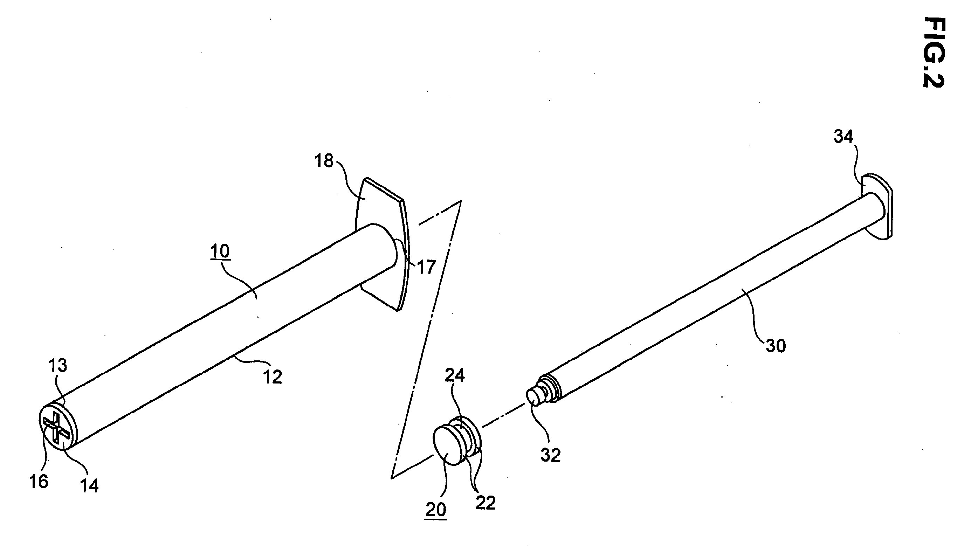

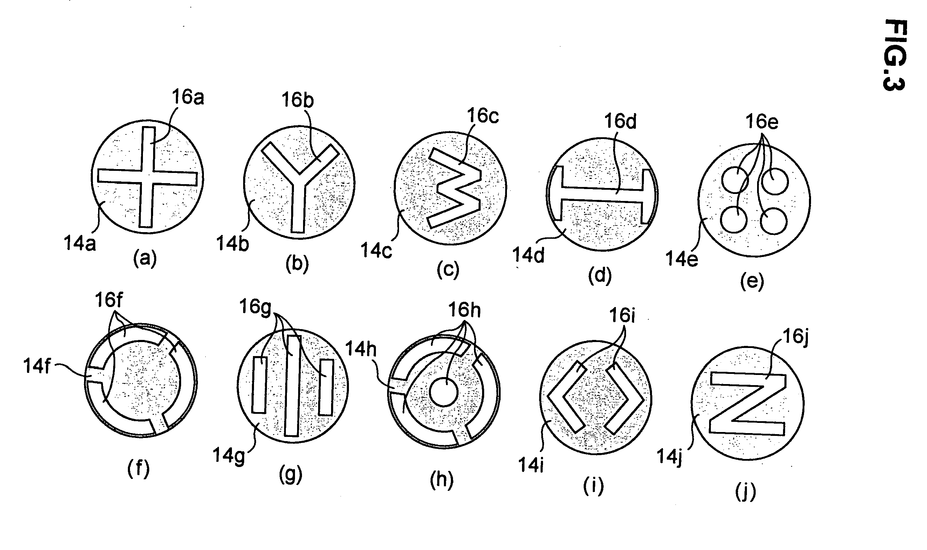

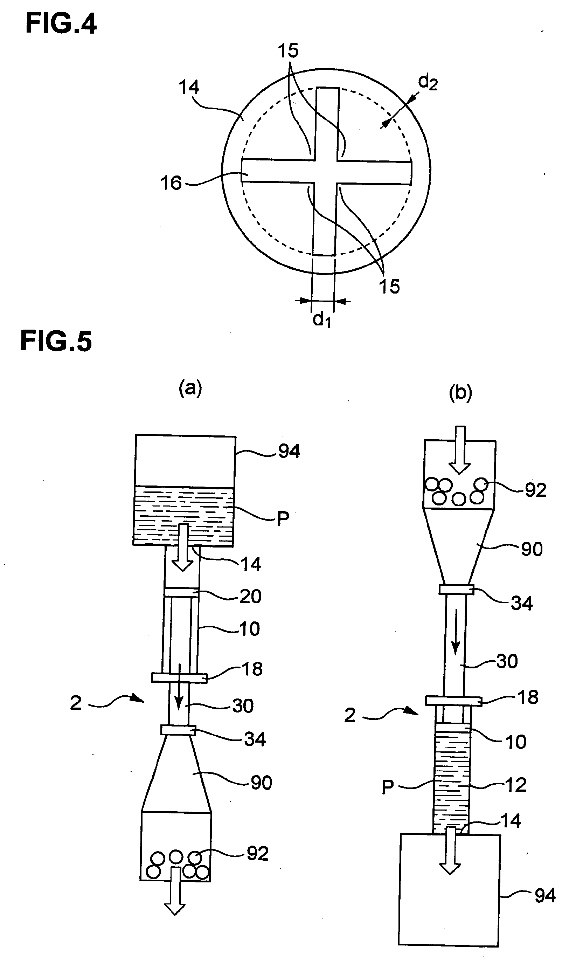

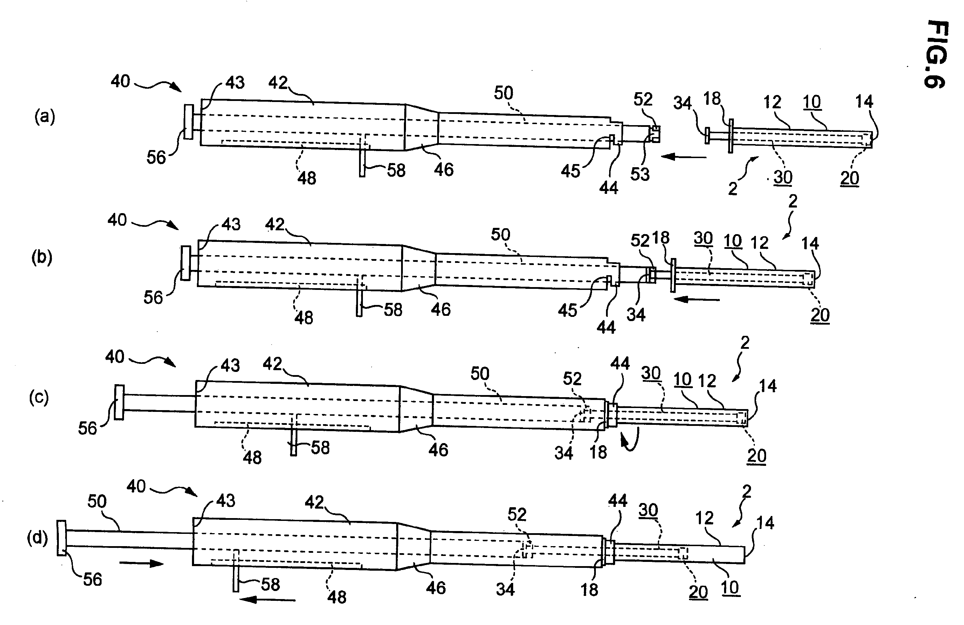

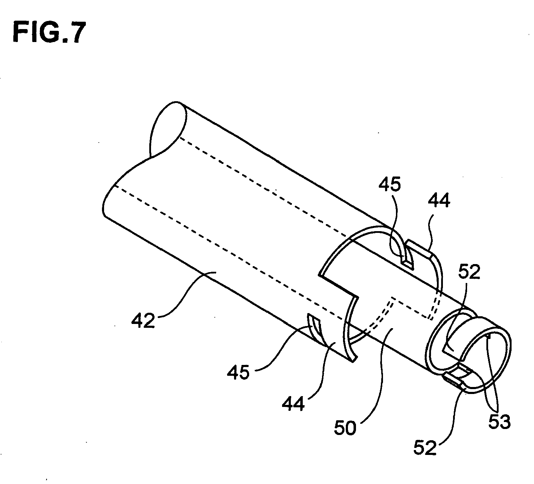

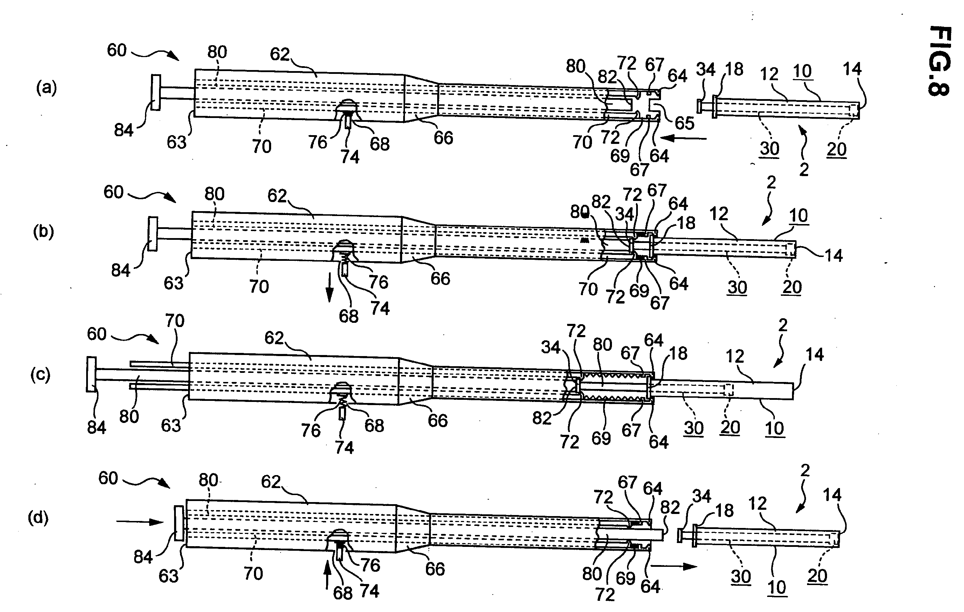

Fig. 1 is a perspective view showing a sampling device for a viscous sample according to the present invention. Fig. 2 is an exploded perspective view shown in Fig. 1. Fig. 3 is a plan view showing various examples of the shape of a slit (aspiration and discharge port) of a plate-like nozzle in the present invention. Fig. 4 is an enlarged explanatory diagram shown in Fig. 3(a). Fig. 5 is a schematic explanatory diagram of the device of the present invention used in measuring aspiration power and discharge power, with (a) illustrating the case of aspiration and (b) illustrating the case of discharge powder. Fig. 6 is an explanatory side view of an example of the auxiliary jig for the sampling device of the present invention. Fig. 7 is an enlarged perspective view of a main part of the auxiliary jig shown in Fig. 6(a). Fig. 8 is an explanatory side view of another example of the auxiliary jig for the sampling device of the present invention. Fig. 9 is an enlarged view of a main part of the auxiliary jig shown in Fig. 8(a). In the drawings, reference numeral 2 indicates a sampling device for a viscous sample, reference numeral 40 indicates an example of an auxiliary jig for the sampling device, and reference numeral 60 indicates another example of the auxiliary jig for the sampling device.

The sampling device 2 for a viscous sample according to the present invention, which is of a basic construction that is in a form similar to that of a so-called conventional syringe 110, includes a tubular body 10, a gasket 20, and a plunger 30 (see Figs. 1 and 2). Note that the viscous sample is not particularly limited as far as it is a liquid sample having a high viscosity that can be a subject of examination, analysis or test. The device of the present invention is useful particularly when the viscous sample is sputum, so the description below is made mainly for the case where sputum is used as the viscous sample.

The tubular body 10 has a hollow long straight section 12 with its distal end forming a closed part 13 and its proximal end being an opening 17, and a flange 18 around the periphery of the proximal end thereof (see Figs. 1 and 2). The shape of the flange 18 may be circular, elliptical or square and is not particularly limited. In the illustrated example, the flange 18 has a frusto-elliptical shape obtained by cutting off both ends of an ellipse (see Figs. 1 and 2). However, the point is that the flange has a suitable structure from the viewpoint of engagement or fitting with the auxiliary jig 40, 60 described hereinafter.

The closed part 13 in the distal end of the tubular body 10 is closed with a plate-like nozzle 14 (see Figs. 1 and 2). That is, while in the conventional syringe 110, the distal end has a small-diameter nozzle 114 protruding therefrom (see Fig. 11), the present invention is provided with a plate-like nozzle in place thereof. The plate-like nozzle 14 has approximately the same diameter as that of the straight section 12 of the tubular body. The plate-like nozzle 14 may be either integrally molded with the straight section 12 or separately molded and then externally or internally fitted with the straight section 12. The plate-like nozzle 14 abuts approximately the whole surface of the lower end of the gasket 20, so the viscous sample will not remain upon discharging the viscous sample, thus avoiding the problem that the viscous sample could remain inside the projecting nozzle 114 as in the conventional syringe 110.

The plate-like nozzle 14 is provided by punching with a slit 16 that has a predetermined shape forming an aspiration and discharge port. The predetermined shape of the slit 16 may include various shapes. For example, various shapes as shown in Fig. 3 can be adopted. Conditions for preferable shapes of the slit include, for example, the position of the slit being located near the central part of the plate-like nozzle 14, and the slit having a shape having at least one corner part 15 from the viewpoints of reducing aspiration power and discharge power, and of improving clear-cut separation of the viscous sample while the viscous sample is being aspirated or discharged and so on (see Figs. 3 and 4). The most preferable slit shape is any one of a cross (X-shape), Y-shape, or W-shape.

From the viewpoints of reducing aspiration power and discharge power, improving clear-cut separation of the viscous sample at the time of aspiration and discharge thereof, ease of production, and the like, the slit has a width, referred to as d1, of preferably 0.5 mm to 2.0 mm, more preferably 0.5 mm to 1.5 mm (see Fig. 4). A distance of the slit from the outer periphery of the nozzle, referred to as d2, is preferably 0.5 mm to 2 mm, more preferably 1 mm to 1.8 mm (see Fig. 4).

The material of the tubular body 10 is not particularly limited as far as it is a transparent or translucent material in view of visibility of the inside thereof and may be a glass material. However, from the viewpoints of production cost and processability, synthetic resin materials having transparency, for example, polyvinyl chloride, olefin-based synthetic resin materials such as polyethylene and polypropylene, styrene-based synthetic resin materials such as polystyrene, and other synthetic resin materials can be used for constructing the tubular body 10.

The gasket 20 is internally fitted slidably in contact with the inner wall of the tubular body 10 in the longitudinal direction with respect to the tubular body 10. The gasket 20 may be similar to the conventional syringe and the shape and material of the gasket are not particularly limited. The shape of the gasket must meet the requirement that the gasket has adhesion to the inner wall of the tubular body 10 and abuts a lower end surface thereof with respect to the plate-like nozzle 14. In the example illustrated in the drawings, the gasket has a sealing rim 22 formed on both ends of the outer periphery of a core 24. The lower end surface of the gasket is made flat so as to conform to the shape of the plate-like nozzle 14 (see Figs. 1 and 2). To ensure adhesion (sealing property), the gasket 20 is desirably made of a material having elasticity, for example, an elastomer material, examples of which include: various rubber materials such as natural rubber, butyl rubber, isoprene rubber, butadiene rubber, styrenebutadiene rubber, and silicone rubber; and various types of synthetic resin materials such as polyurethane-based resin, polyester-based resin, polyamide-based resin, olefin-based resin, and styrene-based resin.

The plunger (pusher) 30 is a long rod-like member for slidably operating the gasket 20 having an engagement convex 32 at its distal end and an operation head 34 at its proximal end. Engaging the engagement convex 32 of the plunger 30 with an engagement concave (not shown) inside the gasket 20 and operating the operation head 34 enables the gasket 20 to be slidably operated in the tubular body 10. The material of the plunger 30 is not particularly limited either. For example, olefin-based and styrene-based synthetic resin materials may be injection molded to produce the plunger.

Then, one example (see Figs. 6 and 7) and another example (see Figs. 8 and 9) of the auxiliary jig used as being attached to the sampling device 2 for a viscous sample according to the present invention are described. The auxiliary jigs 40, 60 of the present invention are used auxiliarily to facilitate the operation of the sampling device 2 of the present invention and also allow indirect operation of the sampling device 2 since it is hygienically unpreferable to operate the sampling device 2 directly in contact with hands in view of the fact that a viscous sample such as sputum is handled by the sampling device 2.

The auxiliary jig 40 for a sampling device according to the present invention includes a long tubular external fitted tube member 42 and an internal fitted shaft member 50 which is slidably and coaxially arranged inside the external fitted tube member 42 (see Figs. 6 and 7).

The external fitted tube member 42 has an engagement section 44 that is engageable with the flange 18 of the tubular body 10 in the sampling device 2 (see Figs. 6 and 7). The engagement section 44 is formed with a notch 45 that engages with the flange 18 of the tubular body 10 (see Fig. 7). The proximal end of the external fitted tube member 42 is formed as an opening 43 and a body section of the external fitted tube member 42 is formed with a tapered section 46 in which a guide groove 48 is bored in the longitudinal direction (see Fig. 6).

The internal fitted shaft member 50 is slidably and coaxially arranged inside the external fitted tube member 42 and has at its distal end an engagement section 52 that is engageable with the operation head 34 of the plunger 30 in the sampling device 2. The engagement section 52 has a recess 53 that fits with the operation head 43 of the plunger 30. An operation lever 58 is provided along the guide groove 48 of the external fitted tube member 42. At the proximal end, a press head 56 is provided.

In the case of the auxiliary jig 40 for the sampling device as such, first the operation head 34 of the plunger 30 of the sampling device 2 is engaged with the engagement section 52 of the internal fitted shaft member 50 (see Fig. 6(a) and(b)), and then the flange 18 of the sampling device 2 and the engagement section 44 of the external fitted tube member 42 are engaged with each other so that they are in mesh with each other, so the sampling device 2 can be attached to the auxiliary jig 40 (see Fig. 6(c)). In case of aspirating a viscous sample, the operation lever 58 of the auxiliary jig 40 for a sampling device is pulled toward the proximal end, thus allowing aspiration of a viscous sample by the sampling device 2. In case of discharging the viscous sample, the press head 56 of the auxiliary jig 40 for a sampling device is pressed toward the distal end of the auxiliary jig 40, so the viscous sample can be discharged by the sampling device 2 (see Fig. 6(d)). Therefore, the operator can perform aspiration and discharge of a viscous sample without directly touching the sampling device 2.

On the other hand, the auxiliary jig 60 for a sampling device according to the present invention includes a long external fitted tube member 62, an intermediately fitted tube member 70 slidably and coaxially arranged inside the external fitted tube member 62, and an internal fitted shaft member 80 slidably and coaxially arranged inside the intermediately fitted tube member 70 (see Figs. 8 and 9).

The external fitted tube member 62 is provided on its inner periphery with a fitting convex 64 that is detachably fitted with the flange 18 of the tubular body 10 in the sampling device 2 and also with a locking convex 67 for locking therewith an upper end of the flange 18 when the flange 18 is fitted to the fitting convex 64. Further, the external fitted tube member 62 is formed with an opening 63 at its proximal end and a tapered section 66 inside and a button hole 68 at its body section (see Figs. 8 and 9). Note that when the shape of the flange 18 in the sampling device 2 is formed so that one pair of sides, i.e., front and rear or right and left, is longer than the other pair as in the case of frusto-elliptical (see Figs. 1 and 2), elliptical or rectangular, as shown in the illustrated examples, the external fitted tube member 62 can be provided at its distal end with a notch recess 65 so as to fit with the fitting convex 64 in the direction of the shorter sides of the flange 18.

The intermediate fitted tube member 70 is slidably and coaxially arranged inside the external fitted tube member 62 and is provided, on an inner periphery at its distal end, with a fitting convex 72 that is detachably fitted with the operation head 34 of the plunger 30 in the sampling device 2 (see Figs. 8 and 9). Further, the intermediate fitted tube member 70 is provided with a biasing unit 69 and a locking unit 74 for the external fitted tube member 62. The biasing unit 69 is a spring, which is provided so as to intervene between the distal end of the intermediate fitted tube member 70 and the locking convex 67 of the external fitted tube member 62. The locking unit 74, which is a button that switches locking and release of the intermediate fitted tube member 70 with respect to the external fitted tube member 62, utilizes the biasing power of the spring 76 to make the intermediate fitted tube member 70 lockable with respect to the external fitted tube member 62.

The internal fitted shaft member 80 is slidably and coaxially arranged inside the intermediate tube member 70 and has a distal end serving as an abutting end 82 that abuts the operation head 34 of the plunger 30 in the sampling device 2 and a press head 84 is provided at the proximal end.

In the case of the sampling device 60 as such, first the operation head 34 of the plunger 30 of the sampling device 2 is fitted with the fitting convex 72 of the intermediate fitted tube member 70 (see Fig. 8(a) (b)) and the flange 18 of the sampling device 2 is fitted with the fitting convex 64 of the external fitted tube member 62 (see Fig. 8(a)(b)), so the sampling device 2 can be attached to the auxiliary jig 60 for a sampling device (see Fig. 8(b)). When a viscous sample is to be aspirated, the locking unit 74 is released and the biasing unit 69 is elongated (see Fig. 8(c)) before the aspiration of the viscous sample by the sampling device 2 can be performed. In addition, when a viscous sample is to be discharged, the press head 84 of the auxiliary jig 60 for a sampling device is pressed toward the distal end of the auxiliary jig 60 for the sampling device to enable the discharging of the viscous sample by the sampling device 2 (see Fig. 8(c) and (d)). Further, after the discharging of the viscous sample, the press head 84 is further pressed toward the distal end of the auxiliary jig 60 for the sampling device to thereby enable the sampling device 2 to be detached from the auxiliary jig 60 for a sampling device (see Fig. 8(d)). Therefore, the operator can perform aspiration and discharge of the viscous sample, and in addition, can perform detachment of the sampling device 2, without directly touching the sampling device 2 (see Fig. 8(d)).

Next, the homogenization agent for sputum of the present invention is described.
The homogenization agent for sputum according to the present invention is featured by containing cysteine protease as an active ingredient, capable of treating sputum more quickly under mild conditions and more safely detecting microbes present in the sputum than conventionally, and particularly effective for homogenization treatment of purulent sputum.

The cysteine protease that can be used in the present invention is not particularly limited and preferably includes cysteine proteases derived from plants, more preferably one or more selected from the group consisting of bromelain, papain, chymopapain, and ficin, with bromelain being particularly preferable. These cysteine proteases may be those commercially available or those obtained by a known method.
The above-mentioned bromelain that can be used includes cysteine proteases derived from plants belonging to the Bromeliaceae, more preferably one that is derived from pineapple (Ananas comosus M.).

The homogenization agent of the present invention is not particularly limited as far as it is formulated as a composition that contains cysteine protease as an active ingredient. Usually, the homogenization agent of the present invention is used in the form of an aqueous solution having dissolved cysteine protease in a dissolving liquid such as a buffer solution. The concentration of cysteine protease is not particularly limited and is preferably 0.01 to 1 w/v%, more preferably 0.1 to 0.3 w/v%.

The dissolving liquid having dissolved therein cysteine protease is not particularly limited and buffer solutions having a pH of about 6.0 to about 10 are preferable. The buffer solutions include Tris buffer solution, phosphate buffer solution, carbonate buffer solution and so on. Tris buffer solutions having a pH of about 7 to about 9 are more preferable. Preferably, the buffer solution contains sodium chloride, EDTA and so on. Particularly, it is preferable to use Tris buffer solutions containing sodium chloride as the dissolving liquid. The concentration of sodium chloride in the Tris buffer solution is preferably higher than 0.6% and lower than 1.2% and the concentration of Tris is preferably 10 mM or more and lower than 50 mM. The pH of the Tris buffer solution is preferably 7.0 or more and below 8.0.
The buffer solution may contain other components as far as such do not harm the effects of the present invention.

In the present invention, sputum refers to a mixture of mucin (mucoproteins and mucopolysaccharides) secreted by tracheas and bronchial tubes, and bacteria and dust aspirated from the surrounding, which may contain human cells or blood cells. As for the properties of sputum, sputum is classified into five stages, M1, M2, P1, P2, and P3 according to the classification of Miller & Jones. In particular, purulent sputum of P2 (purulent sputum containing 1/3 to 2/3 of a purulent portion) and P3 (purulent sputum containing 2/3 or more of a purulent portion) contain much purulent portion and the pretreatment takes time accordingly. In particular, P3 is difficult to be homogenized by simple homogenization treatment. The present invention enables quick and simple homogenization treatment of such purulent sputum.

The homogenization method of sputum according to a first aspect of the present invention is characterized by treating sputum with the homogenization agent of the present invention.
In the present invention, the homogenization method for sputum using the homogenization agent is not particularly limited and sputum can be quickly dissolved by mixing the homogenization agent and subject sputum and then allowing the mixture to react at room temperature to 37°C, preferably at room temperature with applying a vortex as appropriate.

In the present invention, microbes in the subject sputum, after the homogenization treatment with the homogenization agent, can be detected after they are cultivated or without cultivation. The method of detecting a microbe is not particularly limited and a wide variety of known methods may be used. In the detection of a microbe, the microbe may be detected directly or the microbe may be detected by detecting a biologically-relevant substance.

In the present invention, the microbe is not particularly limited and examples of the microbe include viruses such as Herpes viruses (HSV, CMV, ZVZ, EBV, HHV, etc.), influenza virus, human immunodeficiency virus (HIV), human T-cell leukemia virus (HTLV), hepatitis viruses (HBV, HCV, HDV, HGV), and other pathogenic viruses for various diseases, such as cold syndromes, gastrointestinal diseases, central nervous system diseases, respiratory diseases, hemorrhagic fever and so on, Rickettsia, bacteria and fungi such as Staphylococcus aureus, Streptococcus, coliform bacteria, Pseudomonas aeruginosa, Legionella, Moraxella, influenza bacteria, Klebsiella, Chlamydia, and mycoplasma.

In the present invention, the biologically-relevant substances include nucleic acids (DNA, RNA), proteins, and peptides and so on. Preparation of DNA or RNA from live cells can be performed by known methods. For example, extraction of DNA can be performed by the method of Blin et al. (Blin et al., Nucleic Acids Res. 3: 2303 (1976)), etc., and extraction of RNA can be performed by the method of Favaloro et al. (Favaloro et al., Methods Enzymol. 65: 718 (1980)), etc. Extraction of rRNA can be performed by dissolving cells with an aqueous sodium hydroxide solution and then neutralizing the resultant solution with hydrochloric acid or the like. Further, the extracted nucleic acids can be detected by using polymerase chain reaction (PCR), hybridization, a PALSAR method (see, for example, Japanese Patent No. 3267576, and Japanese Patent No.3310662), DNA chip, protein chip, and antigen-antibody reaction and so on.

The homogenization method for sputum according to a second aspect of the present invention includes using particles containing one or more members selected from the group consisting of metals and oxides thereof (the particles being referred to herein as metal particles), in which mixing subject sputum in the presence of the particles enables sputum to be more conveniently and more quickly treated under mild conditions and microbes present in the sputum can be detected more stably by stirring subject sputum in the presence of the particles. The homogenization method is particularly effective for homogenization of purulent sputum.

In the present invention, the metal particles contain one or more materials selected from the group consisting of metals and oxides thereof and particles that contain one or more selected from metals and oxides thereof as main components are preferable. The metal particles may contain materials other than the metals and oxides thereof. The particles of the present invention also include those particles whose surface is covered with a thin film of a metal or oxide thereof. When the particles covered with the above-mentioned thin film are used, the material of the particle to be covered is not particularly limited and may be either a metal or a non-metal.
In the present invention, the metal is not particularly limited and examples thereof include iron, aluminum, magnesium, titanium, copper, zinc, nickel, lead, tin, chromium, zirconium, molybdenum, gold, silver, platinum, and alloys containing these as base materials. Iron and stainless steel are particularly preferable. The oxides of the metals are not particularly limited and include oxides of the above-mentioned metals, with alumina being particularly preferable.

The shape of the metal particle is not particularly limited, but spherical particles are preferable. The diameter of the particles is preferably 1 mm or more, and more preferably 4.0 mm or more. The upper limit of the diameter of the particles is not particularly limited and preferably is 10 mm or less. In the present invention, one or more particles of the same type can be used, or two or more types of particles, for example, two or more particles obtained by combining particles different in particle size or material can be used.

Specifically, the homogenization method for sputum using a metal particle preferably involves homogenization by stirring subject sputum mixed with a dissolving liquid such as a buffer solution with the particles. More preferably, sputum is quickly dissolved by mixing subject sputum, sputum pretreating agent, and the particles, and allowing the mixture to react at room temperature to 37°C, preferably at room temperature while applying a vortex as appropriate.
The dissolving liquid is not limited particularly and the dissolving liquids described in the description on the homogenization agent of the present invention can be advantageously used in a similar manner.

As the sputum pretreating agent, known sputum pretreatment agents such as semi-alkaline protease (for example, Sputazyme (manufactured by Kyokuto Pharmaceutical Industrial Co., Ltd.), etc.), NALC-NaOH reagent, a reducing agent, and a surfactant (for example, pretreatment agents described in JP 02-273197 A, WO 02/010744, and Patent Document 4, etc.) and sputum homogenization agent of the present invention containing cysteine protease as an active ingredient can be used. Sputum homogenization agents containing proteolytic enzymes such as semi-alkaline protease or cysteine protease are preferable, and it is particularly preferable to use the above-mentioned homogenization agents of the present invention. These proteolytic enzymes may be used alone or two or more of them may be used in combination.

In the present invention, a microbe present in subject sputum can be detected by treating sputum by the homogenization method using the metal particles, and with or without cultivating the microbe. The method of detecting a microbe is not particularly limited and a wide variety of known methods can be used. In the detection of a microbe, the microbe may be directly detected or the microbe may be detected by detecting a biologically-relevant substance.

The sputum homogenization device of the present invention is a container with a lid that accommodates particles containing one or more members selected form the group consisting of metals and oxides thereof, that is, the above-mentioned metal particles.
Figs. 11 to 14 are schematic explanatory diagrams illustrating first to fourth examples of the sputum homogenization device of the present invention, respectively. In Figs. 11 to 14, 210a to 210d represent a lid of a container, 212a to 212d represent containers, and 214 represents for metal particles.

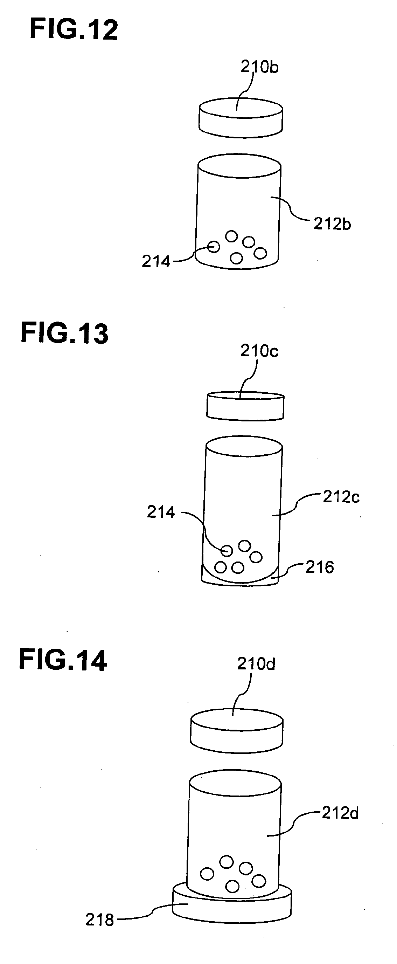

In the sputum homogenization device of the present invention, the shape of the container is not particularly limited and a round-bottomed cylindrical container 212a as shown in Fig. 11, a flat-bottomed cylindrical container 212b as shown in Fig. 12 are preferable. Also, as shown in Fig. 13, a self-standing type cylindrical container 212c having a round bottom inside the container and a flat bottom or cavity at a bottom 216 outside the container may be used. Further, as shown in Fig. 14, a container 212d having a bottom lid 218 attached on the bottom of a cylindrical tube may be used.
The material of the container is not particularly limited and for example, plastic materials such as polyethylene, polypropylene, polystyrene, polyvinyl chloride, and polycarbonate are preferably used.
The lid is not particularly limited as far as it can seal-up the container. The method of attaching the lid is not particularly limited and examples thereof include a cap type method, an embedded type method, an inner screw type method, and an outer screw type method.

As the particle, the above-mentioned metal particles can be used similarly. In this case, spherical particles that are made of stainless steel, iron or alumina are preferable. The particle has a diameter of preferably 1 mm or more, more preferably 4.0 mm or more. The upper limit of the diameter of these particles is not particularly limited and a diameter of 10 mm or less is suitable.
The number of particles contained in the container is not particularly limited and may be determined as appropriate depending on the sizes of the container and the particle.
By performing the sputum homogenization method of the present invention as mentioned above using the sputum homogenization device of the present invention, sputum can be homogenized quickly and easily.

Hereinafter, the method of collecting a microbe according to the present invention is described.
The method of collecting a microbe according to the present invention includes filtering homogenized sputum through a separation device for separating a microbe having at lest three types of filters that allow passage of subject microbe and are made of an organic material, and collecting the subject microbe from the obtained filtrate.

In the method of collecting a microbe according to the present invention, homogenized sputum is used. The homogenization method for sputum is not particularly limited and a wide variety of known methods may be used and the above-mentioned homogenization method of the present invention is preferable.
The sputum homogenization method is preferably a method in which subject sputum is treated with, for example, a dissolving liquid containing the pretreatment agent such as a homogenization agent. As the pretreatment agent and dissolving liquid, the sputum pretreatment agent and the dissolving liquid described in the homogenization method according to the second aspect of the present invention can be preferably used.

The sputum homogenization method using the above-mentioned homogenization agent is not particularly limited and sputum can be quickly dissolved by mixing the dissolving liquid containing the homogenization agent and subject sputum and then allowing the mixture to react at room temperature to 37°C, preferably at room temperature with applying a vortex as appropriate.
In addition, it is preferable that the subject sputum mixed with a dissolving liquid be stirred using particles to homogenize the subject sputum. The particle is not particularly limited and a spherical particle having a diameter of preferably 1 mm or more, more preferably 4 mm or more is suitable. The material of the particle is not particularly limited. A particle that contains one or more members selected from the group consisting of metals and oxides thereof is preferable. Particularly suitable examples of the metals and oxides thereof include stainless steel, iron or alumina.
The homogenization reaction can be carried out in a separation device for separating a microbe.

The homogenized liquid of sputum after the above-mentioned homogenization treatment is filtered using the separation device for separating a microbe according to the present invention.

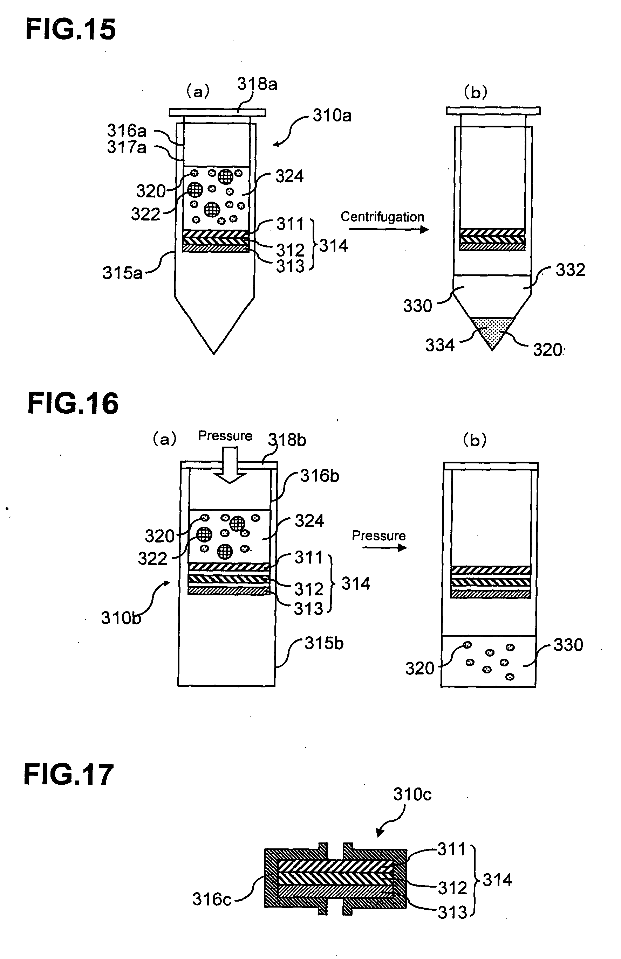

Fig. 15 is a schematic explanatory diagram illustrating one example of the method of collecting a microbe using the microbe separation device of the present invention. In Fig. 15, a separation device 310a for separating a microbe according to the present invention includes a filter member 314 and a filter holder 316a that holds the filter member 314. The filter member 314 includes at least three filters, i.e., a first filter 311, a second filter 312, and a third filter 313, in order in which the homogenized sputum is passed.

The first to third filters are made of a porous material composed of an organic material that allows passage of a subject microbe therethrough. The material of each filter may be the same or different from one another. Note that an example of three-layered structure having each filter laminated is shown in Fig. 15, but the arrangement of filters is not particularly limited and the filters may be arranged keeping a certain interval from one another or laminated.

The organic material is not specifically limited, but preferably an organic polymer fiber, for example: cellulose derivative such as cellulose, cellulose fiber, nitrocellulose, and cellulose acetate; polyolefine such as polyethylene (PE), polypropylene (PP), polystyrene (PS), and cyclopolyolefine; polyamide such as nylon-6, nylon-6,6, nylon-11, nylon-12, and copolyamide; alamido such as polyparaphenylene terephthalamid; polyester such as polyethylene terephthalate (PETP); acryl polymer such as polyacrylnitrile and acrylate; vinyl polymer such as polyvinyl chloride (PVC) and polyvinyl alcohol; polyester ether such as polyether ether ketone (PEEK); polyurethane; epoxide; fluororesin such as polyvinylidine difluoride (PVDF), poly-tetrafluoroethylene (PTFE), poly-hexafluoroethylene/propylene copolymer (FEP), polyethylene/tetrafluoroethylene copolymer (ETFE), and polyethylene/chlorotrifluoroethylene copolymer (ECTFE); polycarbonate; polyphenylene sulfide (PPS); and polyether sulfone. Of those, polycarbonate, polytetrafluoroethylene, polyethylene, cellulose and polyamide are specifically preferable. Those organic materials may be used alone or two or more kinds thereof may be used in combination.

The pore size of the first to third filters is not particularly limited as far as the filters can pass the subject microbe and can be selected as appropriate depending on the size of the subject microbe and the state of the subject sputum.
The third filter is preferably one having a pore size that does not allow passage of cells or blood cells in the sputum but allows passage of the subject microbe. Specifically, the pore size of the third filter is preferably 0.1 µm or more and 10 µm or less, more preferably 2 to 7 µm, still more preferably about 5 µm. The second filter preferably has a pore size larger than that of the third filter. Specifically, the pore size of the second filter is preferably 5 µm or more, more preferably 10 to 30 µm, still more preferably about 20 µm. Preferably, the first filter has a pore size larger than that of the second filter. Specifically, the pore size of the first filter is preferably 5 µm or more, more preferably 20 µm or more, still more preferably 80 to 120 µm.
The thickness of the first to third filters is not particularly limited and preferably is 1 to 200 µm.

The filter holder 316a, for example, as shown in Fig. 15, includes a body 317a that accommodates the filter member 314 and a lid 318a. The shape of the filter holder 316a is not particularly limited and for example, a container that is of a cylindrical shape to which the filter can be attached on the bottom thereof as shown in Fig. 15 can be used. The method of mounting the filter and the method of attaching the lid are not particularly limited and for example, a method in which the filter may be formed integral to the body of the filter holder, and various arranging methods such as a cap type method, an embedded type method, an inner screw method, and an outer screw method can be used.
The material of the filter holder 316a is not particularly limited and examples of the preferable material include plastic materials such as polyethylene, polypropylene, polystyrene, polyvinyl chloride, polycarbonate, and fluororesins.

As shown in Fig. 15(a), a homogenate 324 of sputum after the homogenization treatment frequently contains a subject microbe 320, and a foreign substance 322 such as human-derived cells, blood cells, and dust in admixture. Filtering the homogenate 324 using the separation device 310a of the present invention enables the foreign substances 322 such as the cells, blood cells and dust to be removed with the filter member 314 to provide a filtrate 330 containing the subject microbe 320 (see Fig. 15(b)). In Fig. 15, filtration operation is performed by centrifugation to obtain a filtrate 332 separated into precipitation 334 containing the subject microbe 320 and a supernatant 330. In the present invention, however, the filtration method is not particularly limited. For example, it is preferable that the filtration treatment be carried out using nitrogen gas or syringe under pressure or under reduced pressure conditions.

In the present invention, the homogenate obtained by homogenizing sputum by the above-mentioned homogenization method may be injected into a separation device of the present invention and passed through a separation member to collect the filtrate. Alternatively, the filtrate can be obtained by charging a subject sputum, a dissolving liquid and a pretreatment agent in the separation device of the present invention, homogenizing the sputum in the separation device, and then passing the homogenate through the separation member, and collecting the filtrate.

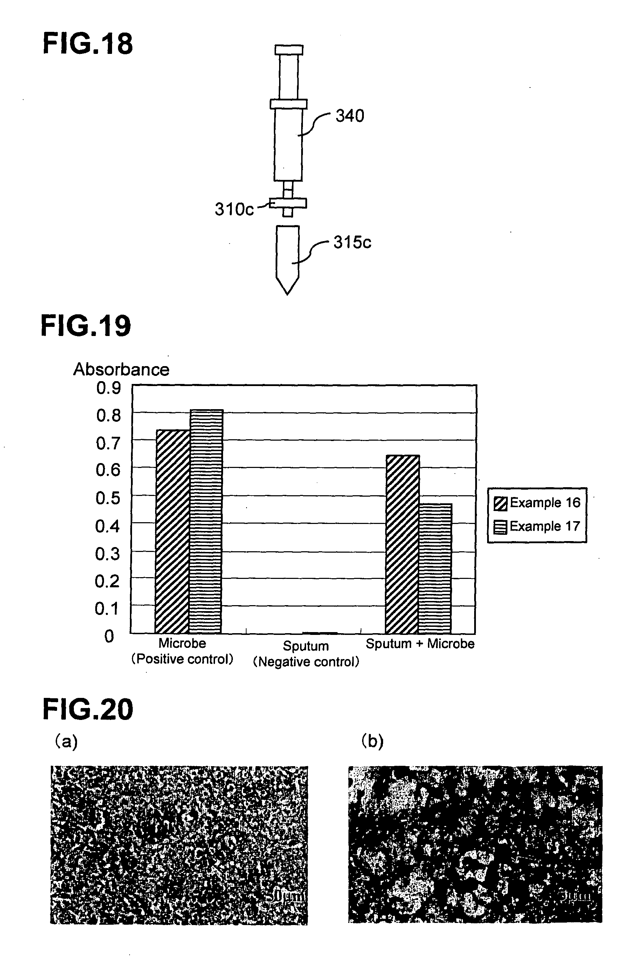

In Fig. 15, 315a represents a collecting container that collects the filtrate 330. In the present invention, the collecting container 315a is not particularly limited as far as the collecting container 315a can collect the filtrate and may be arranged in the separation device as shown in Fig. 15 and 16 or may be separate from the separation device as shown in Figs. 17 and 18. The shape of the collecting container is not particularly limited and for example, a container that is cylindrical and has an inner bottom in the form of a round bottom, a V-shaped bottom, or a flat bottom may be used. The material of the collecting container 315a is not particularly limited and may preferably be a plastic material such as polyethylene, polypropylene, polystyrene, polyvinyl chloride, polycarbonate or a fluororesin.

Fig. 16 is a schematic explanatory diagram illustrating another example of the method of collecting a microbe using the separation device for separating a microbe according to the present invention. In Fig. 16, 310b represents a separation device for separating a microbe according to the present invention, which is an example of use of pressurized filtering. As shown in Fig. 16, the homogenate 324 is charged in the filter holder 316b that holds the separation member 314 and pressure is applied from above using nitrogen gas or the like, so the filtrate 330 containing the subject microbe 320 can be collected in the collecting container 315b. The method of applying pressure is not particularly limited and for example, a method in which a pressurization means (not shown) is connected to the lid 318b of the filter holder 316b, a method in which an air hole (not shown) is provided to the lid 318b and pressure is applied through the air hole, or the like may be used.

Fig. 17 is a schematic cross-sectional explanatory view showing an example of the separation device of the present invention. Fig. 18 shows an example of a kit for collecting a microbe including the separation device of the present invention shown in Fig. 17. In Fig. 17, a separation device 310c of the present invention includes a filter holder 316c, in which a separation member 314 having the first to third filters 311 to 313 is accommodated. As shown in Fig. 18, a syringe 340 is connected to the filter holder 316c and the homogenate is charged therethrough into the syringe 340. Then, with the use of the syringe 340, filtration is performed under pressure. The obtained filtrate is collected in a collecting container 315c, thus providing a filtrate that contains the subject microbe.

In the present invention, the obtained microbe-containing liquid is detected for a microbe after cultivation of the microbe or without cultivation of the microbe, so the microbe present in the subject sputum can be detected. The method of detecting a microbe is not particularly limited and a wide variety of known methods can be used. In detecting a microbe, the microbe may be directly detected or the microbe may be detected by detecting a biologically-relevant substance.

### Examples

Hereinafter, the present invention will be described in more detail by way of examples. However, these examples are merely exemplary and the present invention should not be considered as limitative.

### (Example 1)

As a viscous sample resembling sputum, 8g of pig stomach-derived mucin (manufactured by Wako Pure Chemical Industries, Ltd.) was sufficiently dissolved in 10 ml of 0.9% of sodium chloride solution to prepare 80% of mucin solution, which was used in the following procedure. As the sampling device 2 of the present invention, plate-like nozzles 14a to 14j having various types of slits (aspiration and discharge ports) 16a to 16j as shown in Fig. 3(a) to (j) were used for examining aspiration power and discharge power, respectively. The width of each slit was set to 1 mm and the volume was set to 1 ml. The testing apparatus as shown in Fig. 5 was used. Fig. 5 is a schematic explanatory diagram showing the apparatus used in measuring aspiration power and discharge power according to the present invention. Fig. 5(a) indicates the case of aspiration power and Fig. 5(b) indicates the case of discharge power. In Fig. 5(a) and (b), symbol P represents a viscous sample, numeral 90 represents a weight holder, numeral 92 represents a weight, and numeral 94 represents a sample container. In Fig. 5(a), a sample container 94, which is arranged upside, is charged with the viscous sample P. The lower end of the sample container 94 is connected to the plate-like nozzle 14 of the sampling device 2 of the present invention, the weight holder 90 is suspended from the operation head 34 of the plunger 30 in the sampling device 2 of the present invention and connected thereto. Aspiration power was measured by gradually increasing the weight 92. In Fig. 5(b), the sample container 94 was arranged downside and the plate-like nozzle 14 of the sampling device 2 of the present invention after aspirating the viscous sample P was connected to the upper end of the sample container 94. The weight holder 90 was mounted on and connected to the operation head 34 of the plunger 30 of the sampling device 2 and discharge power was measured by gradually increasing the weight 92. Iron particles (diameter: 6.4 mm) were used as the weight 92. Aspiration power and discharge power were calculated using the formula: F=mg (N). The results obtained are shown in Table 1.

### (Comparative Example 1)

Experiments were conducted in the same manner as that in Example 1 except that a commercially available syringe for tuberculin test manufactured by Terumo Corporation (volume 1 ml) was used as the sampling device. The results obtained are shown in Table 1.

**[Table 1]**

| | Type of slit shape | Aspiration power | Discharge power |
|---|---|---|---|
| | | (F=mg(N)) | (F=mg(N)) |
| | (a) | 4.0x10⁻¹ | 3.7x10⁻¹ |
| | (b) | 3.9x10⁻¹ | 4.0x10⁻¹ |
| | (c) | 4.1x10⁻¹ | 4.3x10⁻¹ |
| | (d) | 4.8x10⁻¹ | 4.8x10⁻¹ |
| Example 1 | (e) | 4.5x10⁻¹ | 4.7x10⁻¹ |
| | (f) | 4.4x10⁻¹ | 4.9x10⁻¹ |
| | (g) | 4.4x10⁻¹ | 4.8x10⁻¹ |
| | (h) | 4.4x10⁻¹ | 4.8x10⁻¹ |
| | (i) | 4.8x10⁻¹ | 5.0x10⁻¹ |
| | (j) | 4.0x10⁻¹ | 4.0x10⁻¹ |
| Comparative Example 1 | - | 6.5x10⁻¹ | 6.4x10⁻¹ |

As shown in Table 1, in Comparative Example 1 in which a commercially available syringe for tuberculin test manufactured by Terumo Corporation (volume 1 ml) was used as the sampling device, both the aspiration power and the discharge power were found to be high in numerical value. In contrast, the sampling device 2 of the present invention using the plate-like nozzles 14a to 14j having slits 16a to 16j of various shapes as shown in Fig. 3(a) to (j) showed both aspiration power and discharge power of lower levels. In particular, with the slits having a cross-shape (X-shaped), Y-shaped, and W-shaped as shown in Fig. 3(a) to (c) and the slit having a Z-shape as shown in Fig. 3(j) showed particularly good results. The results confirm that use of the sampling device 2 enables aspiration and discharge of a viscous sample such as sputum with a relatively small force, so a burden to an operator can be reduced.

### (Example 2)

Using purulent sputum (P2, according to the classification of Miller & Jones) as the viscous sample and with the sampling device 2 of the present invention equipped with plate-like nozzles 14a to 14j having slits (aspiration and discharge port) of various shapes 16a to 16j as shown in Fig. 3(a) to (j), viscosity length (length of strings of a viscous sample) after aspiration and after discharge were examined. The width of each slit was set to 1 mm and the volume was set to 1 ml. The viscosity length after aspiration is determined as follows: after a predetermined amount of a viscous sample was aspirated from the container containing the viscous sample using the sampling device 2 of the present invention, the distal end of the sampling device 2 was once brought in contact with the bottom of the container and then drawn until a string of the viscous sample formed was broken. A distance from the bottom of the container to the height of the distal end of the sampling device 2 at which the string of the viscous sample was broken is referred to as a viscosity length after aspiration. On the other hand, viscosity length after discharge is determined as follows: a predetermined amount of the viscous sample aspirated using the sampling device 2 of the present invention was once totally discharged onto the bottom of the container and then was drawn up until the string of the viscous sample was broken. A distance from the bottom of the container to a height at which the string of the viscous sample was broken is referred to as viscosity length after discharge. The results obtained are shown in Table 2.

### (Comparative Example 2)

Experiments were conducted in the same manner as that in Example 2 except that a commercially available syringe for tuberculin test manufactured by Terumo Corporation (volume 1 ml) was used as the sampling device. The results obtained are additionally shown in Table 2.

**[Table 2]**

| | Type of slit shape | Viscosity length after aspiration | Viscosity length after discharge |
|---|---|---|---|
| | | (cm) | (cm) |
| | (a) | 4.5 | 4.5 |
| | (b) | 4.5 | 4.8 |
| | (c) | 4.5 | 4.5 |
| | (d) | 5.0 | 5.3 |
| Example 2 | (e) | 5.3 | 5.8 |
| | (f) | 5.5 | 5.8 |
| | (g) | 5.5 | 5.8 |
| | (h) | 5.3 | 5.8 |
| | (i) | 5.5 | 6.0 |
| | (j) | 5.0 | 5.3 |
| Comparative Example 2 | - | 5.5 | 6.0 |

As shown in Table 2, in Comparative Example 2 in which a commercially available syringe for tuberculin test manufactured by Terumo Corporation (volume 1 ml) was used as the sampling device 2, the viscosity length after aspiration and after discharge are long. On the contrary, the sampling device 2 with plate-like nozzles 14a to 14j having various types of slits 16a to 16j as shown in Fig. 3(a) to (j) showed viscosity length after aspiration and after discharge equal to or shorter than the value of Comparative Example 2. In particular, considerably good results were obtained with the slits having a cross-shape (X-shaped), Y-shaped, and W-shaped as shown in Fig. 3(a) to (c). In addition, the I-shaped slit shown in Fig. 3(d) and the Z-shaped slit shown in Fig. 3(j) gave second-best results. As the tendency of slit shapes providing clear-cut separation (short viscosity length) of the viscous sample at the time of aspiration and discharge, it revealed that the slit 16 is positioned near the center of the plate-like nozzle 14 and that the slit has at least one corner 15. The sampling device 2 of the present invention can provide clear-cut separation of a viscous sample at the time of aspiration and discharge and hence is preferable from the viewpoint of environmental sanitation.

### (Example 3)

Using purulent sputum (P2, according to the classification of Miller & Jones) as the viscous sample and with the sampling device 2 of the present invention equipped with plate-like nozzles 14a to 14j having slits (aspiration and discharge port) of various shapes 16a to 16j as shown in Fig. 3(a) to (j), 500 µl each of the viscous sample was collected (aspirated and discharged in a separate container) and weight of the samples was measured. This procedure was repeated twice to determine quantitativeness of the test. The results obtained are shown in Table 3.

### (Comparative Example 3)

Experiments were conducted in the same manner as that in Example 3 except that a commercially available syringe for tuberculin test manufactured by Terumo Corporation (volume 1 ml) modified by simply cutting the distal end of the tubular body to make an opening serving as an aspiration and discharge port having the same diameter as the straight body section was used as the sampling device. The results obtained are additionally shown in Table 3.

**[Table 3]**

| | Type of slit shape | Weight (g) | |
|---|---|---|---|
| | | First | Second |
| | (a) | 0.50 | 0.50 |
| | (b) | 0.50 | 0.51 |
| | (c) | 0.52 | 0.52 |
| | (d) | 0.51 | 0.51 |
| Example 3 | (e) | 0.51 | 0.52 |
| | (f) | 0.50 | 0.50 |
| | (g) | 0.49 | 0.50 |
| | (h) | 0.49 | 0.49 |
| | (i) | 0.50 | 0.50 |
| | (j) | 0.51 | 0.50 |
| Comparative Example 3 | - | 0.47 | 0.16 |

As shown in Table 3, in Comparative Example 3 in which a commercially available syringe for tuberculin test manufactured by Terumo Corporation (volume 1 ml) modified by cutting the distal end of the tubular body to make an opening serving as an aspiration and discharge port having the same diameter as the straight body section was used, a part of the viscous sample was drawn after the aspiration to deteriorate quantitativeness. On the contrary, with the sampling device 2 using plate-like nozzles 14a to 14j having various types of slits 16a to 16j as shown in Fig. 3(a) to (j), stable high quantitativeness was exhibited both in the first and second tests. Therefore, it was confirmed that the shape of the aspiration and discharge port of the sampling device is important for quantitativeness and the sampling device 2 of the present invention can remarkably improve the quantitativeness when sampling viscous samples.

### (Example 4)

Using purulent sputum (P2, according to the classification of Miller & Jones) as the viscous sample and with the sampling device 2 of the present invention equipped with a plate-like nozzle 14a having a slit (aspiration and discharge port) having a shape of 16a (cross-shape to X-shape) as shown in Fig. 3(a) with the width of slit d1 set to 0.5 mm, 1 mm, 1.5 mm or 2 mm, viscosity lengths after aspiration and after discharge were examined. The viscosity length after aspiration is determined as follows: after a predetermined amount of a viscous sample was aspirated from the container containing the viscous sample using the sampling device 2 of the present invention, the distal end of the sampling device 2 was once brought in contact with the bottom of the container and then drawn until a string of the viscous sample formed was broken. Then, the distance from the bottom of the container and the height of the distal end of the sampling device 2 at which the string of the viscous sample was broken is referred to as a viscosity length after aspiration. On the other hand, viscosity length after discharge is determined as follows: a predetermined amount of the viscous sample aspirated using the sampling device 2 of the present invention was once totally discharged onto the bottom of the container and then was drawn up until the string of the viscous sample was broken. A distance from the bottom of the container to a height at which the string of the viscous sample was broken is referred to as viscosity length after discharge. The results obtained are shown in Table 4.

### (Comparative Example 4)

Experiments were conducted in the same manner as that in Example 4 except that a commercially available syringe for tuberculin test manufactured by Terumo Corporation (volume 1 ml) was used as the sampling device. The results obtained are additionally shown in Table 4.

**[Table 4]**

| | Width of slit | Viscosity length after aspiration | Viscosity length after discharge |
|---|---|---|---|
| | (mm) | (cm) | (cm) |
| Example 4 | 0.5 | 4.5 | 4.8 |
| | 1.0 | 4.5 | 4.5 |
| | 1.5 | 5.0 | 5.1 |
| | 2.0 | 5.3 | 5.8 |
| Comparative Example 4 | - | 5.5 | 6.0 |

As shown in Table 4, as compared with Comparative Example 4 in which a commercially available syringe for tuberculin test manufactured by Terumo Corporation (volume 1 ml) was used, the cases in which the width of slit in the sampling device 2 of the present invention in which the width of slit d1 was set to 0.5 mm to 2 mm, both the viscosity lengths after aspiration and after discharge were shorter and in particular with the width d1 of the slit set to 0.5 mm to 1.5 mm, good results were obtained. Therefore, it was confirmed that with the sampling device 2 of the present invention, the separation of the viscous sample at the time of aspiration and discharge can be improved and hence preferable in view of environmental sanitation. Note that in the case of the sampling device 2 of the present invention equipped with plate-like nozzles 14b to 14j having slits (aspiration and discharge port) of shapes 16b to 16j as shown in Fig. 3(b) to (j), substantially the same results were obtained.

### (Example 5)

Using glycerol (manufactured by Kishida Chemical Co., Ltd.), ethylene glycol (manufactured by Kishida Chemical Co., Ltd.), Triton X-100 (manufactured by Kishida Chemical Co., Ltd.), or Tween 20 (manufactured by Sigma Corporation) as types of viscous samples and with the sampling device 2 of the present invention equipped with a plate-like nozzle 14a having slits (aspiration and discharge port) of various shapes (cross-shaped to X-shaped) 16a as shown in Fig. 3(a), 500 µl each of the viscous sample was collected (aspirated and discharged in a separate container) and weight of the samples was measured. This procedure was repeated twice to determine quantitativeness depending on types of viscous samples. The results obtained are shown in Table 5. Note that theoretical values calculated from specific gravity of each sample (glycerol: 1.26, ethylene glycol: 1.1088, Triton X-100: 1.07, Tween 20: 1.105) are shown in Table 5 in addition.

**[Table 5]**

| Results of Example 5 | | | |
|---|---|---|---|
| Type of viscous sample | Weight (g) | | Theoretical value (g) |
| | First | Second | |
| Glycerol | 0.63 | 0.63 | 0.63 |
| Ethylene glycol | 0.53 | 0.53 | 0.54 |
| Triton X-100 | 0.55 | 0.55 | 0.55 |
| Tween 20 | 0.56 | 0.56 | 0.55 |

The results in Table 5 confirm that the sampling device 2 of the present invention provides high quantitativeness for various types of viscous samples. Note that in the case of the sampling device 2 of the present invention equipped with plate-like nozzles 14b to 14j having slits (aspiration and discharge port) of shapes 16b to 16j as shown in Fig. 3(b) to (j), approximately the same results were obtained.

As described above, according to the present invention, there can be provided a sampling device 2 and an auxiliary jig 40, 60 therefor that improve quantitativeness when sampling a viscous sample such as sputum, reduce a burden to an operator with a relatively low aspiration and discharge powers, makes it easier to separate the viscous sample upon aspiration and discharge of the viscous sample, and give a less problem on environmental sanitation.

### (Example 6)

0. 25% (2000 U/ml) of Bromelain F (manufactured by Amano Enzyme Inc.) was dissolved in a dissolving liquid (25 mM of Tris buffer solution (pH 7.0), 0.9% of sodium chloride, 0.1 mM of sodium ethylene diamine tetraacetate (EDTA)) to prepare a pretreatment liquid. The pretreatment liquid was added to purulent sputum (P2 and P3, according to the classification of Miller & Jones) in a 20-fold amount and the resultant mixture was allowed to react at room temperature (26°C). On this occasion, the reaction mixture was suspended by using a vortex (TM-252 TEST TUBE MIXER, manufactured by Asahi Techno Glass Corporation) after every 10 minutes and dissolution time was measured. In this example and examples to be described later, the dissolution time for sputum is defined as a reaction time that is required for achieving a state of substantial complete liquefaction of sputum. The results obtained are shown in Table 6.

### (Comparative Example 5)

Sputazyme (manufactured by Kyokuto Pharmaceutical Industrial Co., Ltd., semi-alkaline protease) was dissolved in a phosphate buffer dissolving liquid attached to the kit to prepare a pretreatment liquid. Experiments were conducted in the same manner as in Example 6 except that the pretreatment liquid was changed. The results obtained are shown in Table 6.

**[Table 6]**

| | Example 6 | Comparative Example 5 |
|---|---|---|
| P2 | 70 minutes | 120 minutes |
| P3 | 90 minutes | Longer than 180 minutes |

As shown in Table 6, in Comparative Example 5 in which semi-alkaline protease was used, the liquefaction of P2 purulent sputum took 120 minutes and P3 purulent sputum did not liquefy even after reaction for 180 minutes. On the contrary, in Example 6 using bromelain, P2 purulent sputum and P3 purulent sputum were liquefied quickly in 70 minutes and 90 minutes, respectively. Therefore, it revealed that use of bromelain enables quick homogenization to be carried out and further enables quick homogenization of purulent sputum containing much purulent portion that could hardly be homogenized in the conventional manner.

### (Experimental Example 1)

0. 25% (2000 U/ml) of Bromelain F (manufactured by Amano Enzyme Inc.) was dissolved in a dissolving liquid (0.9% of sodium chloride solution, 25 mM of Tris buffer solution (pH 7.0), 0.1 mM of EDTA) to prepare a suspension. To the suspension, 10³ CFU/ml of influenza bacillus (Haemophilus influenzae, H. influenzae), Streptococcus pneumoniae (S. pneumoniae), Pseudomonas aeruginosa (P. aeruginosa), Staphylococcus aureus (S. aureus), Legionella pneumophila (L. pneumophila), or Klebsiella pneumoniae (K. pneumoniae) was added and the mixture was incubated at room temperature (26°C) for 0 hour or 2 hours. The obtained viable cell numbers were compared. Further, as a control, viable cell number was counted under the same conditions as above except that the dissolving liquid without adding bromelain F was used as a suspension.

The viable cell number was obtained by inoculating each of the bacterial suspension on three plates of chocolate agar medium EX (manufactured by Nissui Pharmaceutical Co., Ltd.) in a dose of 100 µl per plate, cultivating the viable cell at 37°C for 18 hours, and counting colony count. An average value of the obtained results was used as a viable cell number. The results obtained are shown in Table 7.

### (Comparative Example 6)

Experiments were conducted in the same manner as that in Example 1 except that Sputazyme (manufactured by Kyokuto Pharmaceutical Industrial Co., Ltd., semi-alkaline protease) was used as the suspension and influenza bacillus was added as the bacterium. The results obtained are shown in Table 8.

**[Table 7]**

| Results of Experimental Example 1 | | | |
|---|---|---|---|
| Bacterium | Enzyme | Viable cell number (CFU/mL) | |
| | | 0 Hour | 2 Hours |
| *H. influenzae* | Bromelain | 2.1x10³ | 1.5x10³ |
| | - | 2.5x10³ | 2.4x10³ |
| *P. aeruginosa* | Bromelain | 4.3x10³ | 4.4x10³ |
| | - | 5.0x10³ | 4.0x10³ |
| *K. pneumoniae* | Bromelain | 3.5x10³ | 4.2x10³ |
| | - | 3.2x10³ | 3.5x10³ |
| *L. pneumophila* | Bromelain | 2.7x10³ | 3.0x10³ |
| | - | 1.6x10³ | 1.7x10³ |
| *S. aureus* | Bromelain | 4.6x10³ | 4.6x10³ |
| | - | 3.9x10³ | 3.6x10³ |
| *S. pneumoniae* | Bromelain | 1.3x10³ | 1.1x10³ |
| | - | 1.3x10³ | 1.0x10³ |

**[Table 8]**

| Results of Comparative Example | | | |
|---|---|---|---|
| Bacterium | Enzyme | Viable cell number (CFU/mL) | |
| | | 0 Hour | 2 Hours |
| *H. influenzae* | Sputazyme | 1.6x10³ | 0.6x10³ |

As shown in Table 7, use of bromelain prevented any of bacteria out of H. influenzae, S. pneumoniae, P. aeruginosa, S. aureus, L. pneumophila, and K. pneumoniae from being affected by enzymes.
Further, in Comparative Example 6 using semi-alkaline protease, reaction at room temperature for 2 hours led to decrease in viable cell number. In contrast, in Experimental Example 1, reaction at room temperature for 2 hours showed no substantial decrease in viable cell number. Therefore, it revealed that use of bromelain enables stable detection of bacteria without affecting bacteria.

### (Examples 7 to 10)

Bromelain F (manufactured by Amano Enzyme Inc., 1000 U/ml) and papain (manufactured by Amano Enzyme Inc., 1000 U/ml) were dissolved in a dissolving liquid (10 mM of Tris buffer solution (pH 7.0), 0.9% of sodium chloride, 0.1 mM of sodium ethylenediamine tetraacetate) to prepare a pretreatment liquid. In a 2-ml Eppendorf tube, sputum (State P3 purulent sputum), 10-fold amount of pretreatment liquid based on sputum, and particles having the material and diameter shown in Tables 9 and 10 were charged and the mixture was allowed to react at room temperature (26°C). On this occasion, the reaction mixture was suspended by using a vortex (TM-252 TEST TUBE MIXER, manufactured by Asahi Techno Glass Corporation) every 10 minutes and dissolution time was measured. The results of measurement of dissolution time are shown in Tables 9 and 10.

The numbers of iron and stainless steel particles were as follows: diameter of 1.6 mm: 40 grains, diameter of 2.4 mm: 20 grains, diameter of 3.2 mm: 10 grains, diameter of 4.0 mm: 7 grains, diameter of 4.8 mm: 4 grains, and diameter of 5.6 mm: 3 grains. The number of alumina particles is as follows: diameter of 1.0 mm: 40 grains, diameter of 2.0 mm: 20 grains, diameter of 3.0 mm: 10 grains, diameter of 4.0 mm: 7 grains, and diameter of 5.0 mm: 4 grains. The number of glass particles is as follows: diameter of 1.5 to 2.5 mm: 20 grains, diameter of 4.0 to 4.7 mm: 7 grains, and diameter of 4.8 to 5.6 mm: 4 grains.

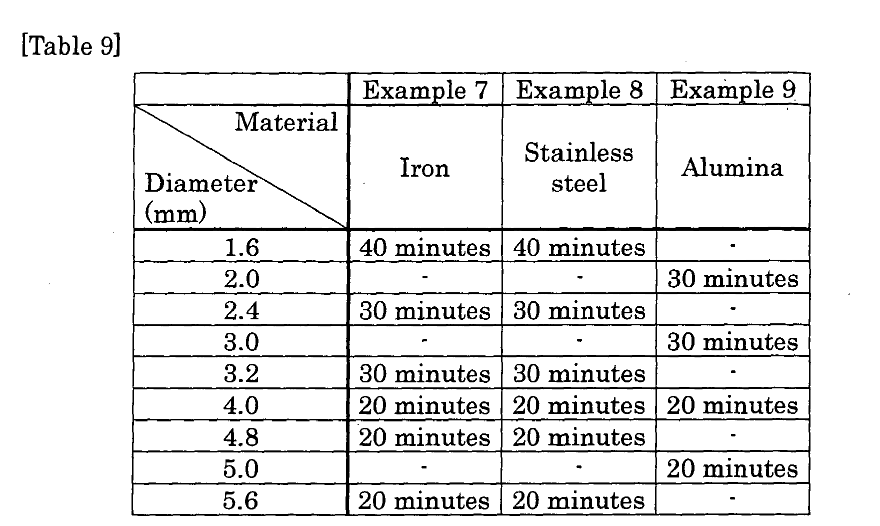

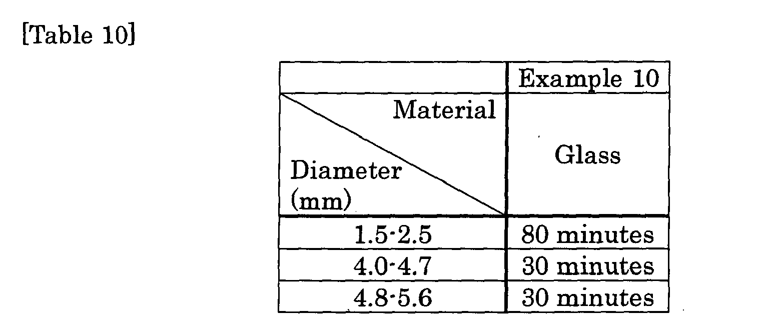

As shown in Tables 9 and 10, the use of iron, stainless steel or alumina particles resulted in a significant reduction in sputum dissolution time. It was found that particles of stainless steel, iron or alumina having a diameter of 4.0 mm or more enables a further reduction in sputum dissolution time.

### (Experimental Example 2)

H. influenzae and S. aureus were each suspended in a suspension (25 mM of Tris buffer solution (pH 7.0), 0.9% of sodium chloride) to prepare bacteria preparations. In a 2-ml Eppendorf tube, 1 ml of the bacteria preparation and 7 grains of stainless steel particles (diameter of 4mm) were charged. The resultant was shaken using a vortex at a rotation speed of 2500 rpm and then viable cell number was measured every 30 seconds and influence on bacteria in respect of each agitation time was examined. The results obtained are shown in Table 11.

**[Table 11]**

| Results of Experimental Example 2 | | |
|---|---|---|
| Stirring time (Second) | Viable cell number (CFU/mL) | |
| | *H. influenzae* | *S. aureus* |
| 0 | 1.5x10³ | 3.5x10³ |
| 30 | 1.5x10³ | 3.3x10³ |
| 60 | 1.7x10³ | 2.3x10³ |
| 90 | 1.5x10³ | - |
| 120 | 1.5x10³ | 3.2x10³ |
| 150 | 1.2x10³ | - |
| 180 | 1.7x10³ | 2.2x10³ |

As shown in Table 11, application of a vortex for 3 minutes caused no influence on bacteria by the particles.

### (Experimental Example 3)

H. influenzae and S. aureus were each suspended in a suspension (25 mM of Tris buffer solution (pH 7.0), 0.9% of sodium chloride) to prepare bacteria preparations. In a 2-ml Eppendorf tube, 1 ml of the bacteria preparation and each of stainless steel particles having respective diameters were charged. The resultant was shaken using a vortex at a rotation speed of 2500 rpm for 3 minutes and then viable cell number was determined and influence on bacteria was examined. Also, as a control, experiments were conducted under the same conditions as above except that no particles were added. The results obtained are shown in Table 12.
Note that the number of stainless steel particles were determined as follows: the weight of the particles were made the same; diameter of 1.6 mm: 40 grains, diameter of 2.4 mm: 20 grains, diameter of 3.2 mm: 10 grains, diameter of 4.0 mm: 7 grains, diameter of 4.8 mm: 4 grains, and diameter of 5.6 mm: 3 grains.

**[Table 12]**

| Results of Experimental Example 3 | | |
|---|---|---|
| Diameter of particle (mm) | Viable cell number (CFU/mL) | |
| | *H. influenzae* | *S. aureus* |
| No particles | 1.2x10³ | 2.0x10³ |
| 1.6 | 0.4x10³ | 2.3x10³ |
| 2.4 | 0.1x10³ | 2.3x10³ |
| 3.2 | 0.2x10³ | 2.2x10³ |
| 4.0 | 1.1x10³ | 2.7x10³ |
| 4.8 | 1.1x10³ | 2.3x10³ |
| 5.6 | 1.0x10³ | 2.3x10³ |

As shown in Table 12, the homogenization treatment using stainless steel particles had significantly small influence on bacteria and in particular, with particle size of 4.0 mm or more, application of a vortex for 3 minutes had no influence on the bacteria by the particles.

### (Examples 11)

0.25% of bromelain F (manufactured by Amano Enzyme Inc.) was dissolved in a dissolving liquid (25 mM of Tris buffer solution (pH 7.0), 0.9% of sodium chloride, and 0.1 mM of sodium ethylenediamine tetraacetate) to prepare a pretreatment liquid. In a 2-ml Eppendorf tube, sputum (State P2 purulent sputum), 10-fold amount of pretreatment liquid based on sputum, and 7 grains of stainless steel particles (diameter of 4mm) were charged and the mixture was allowed to react at room temperature (26°C). On this occasion, the reaction mixture was suspended by using a vortex every 10 minutes and dissolution time was measured. The results of dissolution time are shown in Table 13.

### (Example 12)

Sputazyme (manufactured by Kyokuto Pharmaceutical Industrial Co., Ltd., semi-alkaline protease) was dissolved in a phosphate buffer dissolving liquid attached to the kit to prepare a pretreatment liquid. Experiments were conducted in the same manner as in Example 11 except that the pretreatment liquid was changed. The results obtained are shown in Table 13.

### (Example 13)

As the pretreatment liquid, the same pretreatment liquid as that in Example 11 was used. In a 10-ml-Spitz tube, sputum (State P2 purulent sputum) and 20-fold amount based on sputum of the pretreatment liquid were charged and the resultant mixture was allowed to react at room temperature (26°C). Thereafter, the reaction mixture was suspended by application of a vortex every 10 minutes, and dissolution time was measured. The results of measurement of dissolution time are shown in Table 13.

### (Comparative Example 7)

As the pretreatment liquid, the same pretreatment liquid as that in Example 12 was used. Experiments were conducted in the same condition as that in Example 13 except that the pretreatment liquid was changed. The results of dissolution time are shown in Table 13.

**[Table 13]**

| | Enzyme | Metal particle | Dissolution time |
|---|---|---|---|
| Example 11 | Bromelain | Yes | 20 minutes |
| Example 12 | Sputazyme | Yes | 20 minutes |
| Example 13 | Bromelain | No | 50 minutes |
| Comparative Example 7 | Sputazyme | No | 90 minutes |

As shown in Table 13, use of stainless steel particles made it possible to reduce sputum dissolution time considerably even in a case of taking a long time to dissolve sputum only with an enzyme in terms of sputum dissolution properties.

### (Experimental Example 4 and Comparative Example 8)

S. aureus was adjusted to make 10³ CFU/ml of bacterial suspension with physiological saline. In a syringe filter holder, each filter having a particle size shown in Table 1 and made of a material as shown in Table 1 was introduced for filtration. This was connected to the syringe and the bacterial suspension was passed therethrough. Then, the collected bacterial suspension (filtrate) was inoculated on a plate and viable cells were counted. The viable cell number was determined in the same manner as that in Example 1. Also, as a control, the bacterial suspension that did not pass through the filter was similarly determined for viable cell number. The results obtained are shown in Table 14.

**[Table 14]**

| | Filter | | Viable cell number (CFU/mL) |
|---|---|---|---|
| | Material | Pore size (µm) | *S. aureus* |
| Example 4 | Polycarbonate | 2 | 2.3x10³ |
| Comparative Example 8 | Glass fiber | 2 | 0.58x10³ |
| Control | - | - | 2.2x10³ |

In table 14, polycarbonate represents Cyclo Pore Membrane manufactured by Whatman Co. (hydrophilic polycarbonate membrane, thickness: 7 to 20 µm), and glass fiber represents GMF 150 (thickness: 0.75 mm) manufactured by Whatman Co.

As a result, in Comparative Example 8 using glass fiber, recovery rate of microbe by filtration considerably decreased in contrast to Example 4 using polycarbonate in which recovery rate of microbes was equal to that of the object that was not filtrated. It was found that use of polycarbonate enables efficient collection of microbes.

### (Examples 5 to 7)

S. pneumoniae, P. aeruginosa and K. pneumoniae were each adjusted to make 10³ CFU/ml of bacterial suspension with physiological saline. In a syringe filter holder, each filter having a particle size shown in Table 15 and made of a material as shown in Table 15 was introduced for filtration. This was connected to the syringe and the bacterial suspension was passed therethrough. Then, the collected bacterial suspension (filtrate) was inoculated on a plate and viable cells were counted. The viable cell number was determined in the same manner as that in Example 1. Also, as a control, the bacterial suspension that did not pass through the filter was similarly determined for viable cell number. The results obtained are shown in Table 15.

**[Table 15]**

| | Filter | | Viable cell number (CFU/mL) | | |
|---|---|---|---|---|---|
| | Material | Pore size (µm) | *S. pneumoniae* | *P. aeruginosa* | *K. pneumoniae* |
| Example 5 | PTFE | 5 | 3.9x10³ | 1.6x10³ | 3.0x10³ |
| Example 6 | Cellulose fiber | 5 | 3.9x10³ | 2.5x10³ | 2.4x10³ |
| Example 7 | polycarbonate | 5 | 4.3x10³ | 2.6x10³ | 3.0x10³ |
| Control | - | - | 4.6x10³ | 2.9x10³ | 2.4x10³ |

In Table 15, PTFE represents a POLYFLON (registered trademark of Daikin Industries, Ltd.) filter (thickness: 0.36mm) manufactured by ADVANTEC Co., Ltd., cellulose fiber represents qualitative filter paper (thickness: 0.26 mm) manufactured by ADVANTEC Co., Ltd., polycarbonate represents Cyclo Pore Membrane (hydrophilic polycarbonate membrane, thickness: 7 to 20 µm) manufactured by Whatman Co., Ltd.

The above results indicate that by using a filter made of an organic material such as PTFE, cellulose fiber or polycarbonate, various bacteria can be collected efficiently.

### (Examples 8 to 9)

P. aeruginosa and S. pneumoniae were each adjusted to make 10³ CFU/ml of bacterial suspension with physiological saline. In a syringe filter holder, each filter having a particle size shown in Table 16 and made of a material as shown in Table 16 was introduced for filtration. This was connected to the syringe and the bacterial suspension was passed therethrough. Then, the collected bacterial suspension (filtrate) was inoculated on a plate and viable cells were counted. The viable cell number was determined in the same manner as that in Example 1. Also, as a control, the bacterial suspension that did not pass through the filter was similarly determined for viable cell number. The results obtained are shown in Table 16.

**[Table 16]**

| | Filter | | Viable cell number (CFU/mL) | |
|---|---|---|---|---|
| | Material | Pore size (µm) | *P. aeruginosa* | *S. pneumoniae* |
| Experimental Example 8 | Nylon | 108 | 4.8x10³ | 4.0x10³ |
| Experimental Example 9 | Polyethylene | 112 | 3.5x10³ | 3.9x10³ |
| Control | - | - | 4.8x10³ | 4.5x10³ |

In Table 16, nylon represents nylon mesh sheet manufactured by Sanplatec Corp. and polyethylene represents PE mesh sheet manufactured by Sanplatec Corp.

The above results indicate that by using filters made of nylon or polyethylene, P. aeruginosa and S. pneumoniae can be efficiently collected.

### (Experimental Examples 10 to 12)

### <Separation of microbes from a mixed suspension of microbes and cells by third and second filters>

In Experimental Example 10, P. aeruginosa was adjusted to 10³ CFU/ml of bacterial suspension with physiological saline and further mixed with a bacterial suspension so that PC-14 cell was adjusted to a population of 10⁶ cells/ml.
In a syringe filter holder was mounted the third filter having a pore size of 5 µm and made of polycarbonate used in Experimental Example 7 and on this, the second filter made of nylon having a pore size of 20 µm and made of nylon (manufactured by Sanplatec Corp., nylon mesh sheet) was mounted. The resultant was connected to a syringe and a mixed suspension of cells and microbes was passed therethrough. Then, the collected bacterial suspension was inoculated on a plate and viable cells were counted. Viable cell number was obtained in the same manner as in Experimental Example 1. The results obtained are shown in Table 17.

Further, as a control, filtrate obtained by passing a mixed suspension of the bacterial suspension and cells (Experimental Example 11) or a bacterial suspension (Experimental Example 12) through the third filter without inserting the second filter, and a bacterial suspension that was not passed through filters (control) were determined viable cell numbers in the same manner as above. The results obtained are shown in Table 17.

**[Table 17]**

| | Sample | Third filter (Lower layer) | | Second filter (Upper layer) | | Viable cell number (CFU/mL) |
|---|---|---|---|---|---|---|
| | | Material | Pore size (µm) | Material | Pore size (µm) | *P. aeruginosa* |
| Experimental Example 10 | Bacterium + cell | polycarbonate | 5 | Nylon | 20 | 2.5x10³ |
| Experimental Example 11 | Bacterium + cell | Polycarbonate | 5 | - | - | - |
| Experimental Example 12 | Bacterium | Polycarbonate | 5 | - | - | 2.1x10³ |
| Control | Bacterium | - | - | - | - | 2.4x10³ |

The above results indicate that when the third filter alone was used, clogging with cells occurred to prevent passage through the filter, but by using a filter having a pore size of 20 µm above a filter having a pore size of 5 µm in combination, P. aeruginosa could be efficiently collected in the presence of cells.

### (Experimental Examples 13 to 17)

### <Passability of viscous solution through second and first filters>

Pig stomach-derived mucin (manufactured by Wako Pure Chemical Industry, Ltd.) was dissolved in physiological saline to prepare a 80% viscous solution. To this was added an equal amount of a 0.1% dithiothreitol (DTT) solution dissolved in physiological saline and the resultant mixture was allowed to react at room temperature for 1 hour to obtain a homogenized viscous solution of mucin. In the syringe filter holder was mounted a second filter made of nylon having a pore size of 20 µm as used in Experimental Example 10 and thereon a first filter made of nylon having a pore size of 42, 59, 77 or 108 µm (manufactured by Sanplatec Corp., nylon mesh sheet) was placed (Experimental Examples 13 to 16). This was connected to a syringe and the homogenized viscous solution of mucin was passed therethrough to examine the passability of the filters. In addition, as a control, a syringe filter holder having mounted therein only the second filter was used to examine the passability of the filter in the same manner as above (Experimental Example 17). The results obtained are shown in Table 18.

**[Table 18]**

| | Second filter (Lower layer) | | First filter (Upper layer) | | Filter passability |
|---|---|---|---|---|---|
| | Material | Pore size (µm) | Material | Pore size (µm) | |
| Experimental Example 13 | Nylon | 20 | Nylon | 42 | ○ |
| Experimental Example 14 | Nylon | 20 | Nylon | 59 | ○ |
| Experimental Example 15 | Nylon | 20 | Nylon | 77 | ○ |
| Experimental Example 16 | Nylon | 20 | Nylon | 108 | ⊚ |
| Experimental Example 17 | Nylon | 20 | - | - | Δ |

In Table 18, evaluation standards for the passability of filter are as described below.
⊚: Syringe operation being smoother;
○: Syringe operation being smooth;
Δ: Syringe operation being hard;
x: Syringe operation being considerably hard.

The results shown in Table 18 indicate that with the syringe filter holder having mounted therein only a filter having a pore size of 20 µm (Experimental Example 17), the syringe operation was hard and it took relatively long time for passing the homogenized viscous solution of mucin through the filter while combinations of the filter having a pore size of 20 µm with the filter having a pore size of 42µm, 59µm or 77 µm (Experimental Examples 13 to 15) allowed the homogenized viscous solution of mucin to pass through the filters relatively smoothly. In particular, use of the combination of the filter having a pore size of 20 µm with the filter having a pore size of 108 µm (Experimental Example 16) could achieve smoother passage of the homogenized viscous solution of mucin. This indicates that the size of the first filter is preferably larger than 20 µm, and more preferably about 100 µm.

### (Examples 14 and 15 and Experimental Example 18)

### <Passability of homogenate of sputum through first, second and third filters>

Bromelain F (manufactured by Amano Enzyme Inc.) was dissolved in a dissolving liquid (25 mM of Tris buffer solution (pH 7.0), 0.9% of sodium chloride, and 0.1 mM of sodium ethylenediaminetetraacetate (EDTA)) to a final concentration of 2000 U/ml (0.25%) to prepare a pretreatment liquid. In a 2-ml Eppendorf tube were charged sputum (State P3 purulent sputum), a 10-fold amount based on sputum of the pretreatment liquid, and 7 grains of stainless steel particles having a diameter of 4 mm and the mixture was allowed to react at room temperature (26°C). On this occasion, the reaction mixture was suspended by using a vortex (TM-252 TEST TUBE MIXER, manufactured by Asahi Techno Glass Corporation) every 10 minutes. The solution after 20 minutes of reaction was named a homogenate of sputum.

In a syringe filter holder was charged a third filter having a pore size of 5 µm and made of polycarbonate as used in Experimental Example 7 and thereon a second filter having a pore size of 20 µm and made of nylon as used in Experimental Example 10, and further thereon a first filter having a pore size of 42 µm or 108 µm and made of nylon (manufactured by Sanplatec Corp., nylon mesh sheet) (Example 14 or 15). This was connected to a syringe and the homogenate of sputum was passed through the filters to examine the passability of the filters. Further, as a control, a syringe filter holder having charged therein only the third filter and the second filter was used to examine the passability of the filters in a similar manner (Experimental Example 18). The results obtained are shown in Table 19. Evaluation standards of the passability of filters in Table 19 are the same as those described in Table 18.

**[Table 19]**

| | Third filter (Lower layer) | | Second filter (Middle layer) | | First filter (Upper layer) | | Filter passability |
|---|---|---|---|---|---|---|---|
| | Material | Pore size (µm) | Material | Pore size (µm) | Material | Pore size (µm) | |
| Example 14 | Polycarbonate | 5 | Nylon | 20 | Nylon | 42 | ○ |
| Example 15 | Polycarbonate | 5 | Nylon | 20 | Nylon | 108 | ○ |
| Experimental Example 18 | Polycarbonate | 5 | Nylon | 20 | - | - | × |

The results in Table 19 indicate that with the syringe filter holder having charged therein only two types of filters having a pore size of 5 µm and of 20 µm (Experimental Example 18), the syringe operation was hard and it took relatively long time for passing the homogenized viscous solution of sputum through the filters. On the other hand, combinations of the filters each having a pore size of 5 µm and 20 µm with the filter having a pore size of 42 µm or 108 µm (Experimental Examples 14 or 15) allowed the homogenized solution of sputum to pass through the filters relatively smoothly.

### (Example 16)

### 1. Preparation of bacterial suspension

S. aureus cultivated for 18 hours on tryptic soy agar was suspended in physiological saline to prepare a bacterial culture stock solution. This was diluted with physiological saline to a predetermined number of bacteria to prepare a diluted bacterial suspension used in examples. To obtain the number of microbes, a dilution series of stock bacterial suspension was prepared and cultivated on tryptic soy agar, and viable cell numbers were obtained, from which a predetermined number of cells were calculated. On the other hand, physiological saline instead of the diluted bacterial suspension was similarly reacted and the obtained viable cell number was used as a control.

### 2. Preparation of homogenate of sputum

Bromelain F (manufactured by Amano Enzyme Inc.) was dissolved in a dissolving liquid (25 mM of Tris buffer solution (pH 7.0), 0.9% of sodium chloride, and 0.1 mM of sodium ethylenediaminetetraacetate (EDTA)) to a final concentration of 2000 U/ml (0.25%) to prepare a pretreatment liquid. In a 2-ml Eppendorf tube were charged sputum (State P3 purulent sputum), a 10-fold amount based on sputum of the pretreatment liquid, and 7 grains of stainless steel particles having a diameter of 4 mm and the mixture was allowed to react at room temperature (26°C). On this occasion, the reaction mixture was suspended by using a vortex (TM-252 TEST TUBE MIXER, manufactured by Asahi Techno Glass Corporation) every 10 minutes. The solution after 20 minutes of reaction was named a homogenate of sputum.

### 3. Addition of bacterial suspension

S. aureus cultivated according to the above-mentioned method was diluted with a dissolving liquid (25 mM of Tris buffer solution (pH 7.0), 0.9% of sodium chloride, 0.1 mM of sodium ethylenediaminetetraacetate, 0. 25% (2000 U/ml) of Bromelain F (manufactured by Amano Enzyme Inc.)) so that colony-forming unit was 10×10⁸ CFU/ml and further diluted with the above-mentioned homogenate of sputum so that colony-forming unit was 10×10⁷ CFU/ml.
On the other hand, S. aureus cultivated according to the above-mentioned method was diluted with a dissolving liquid (25 mM of Tris buffer solution (pH 7.0), 0.9% of sodium chloride, 0.1 of mM sodium ethylenediaminetetraacetate, 0. 25% (2000 U/ml) of Bromelain F (manufactured by Amano Enzyme Inc.)) so that colony-forming unit was 10×10⁷ CFU/ml to obtain a positive control.
Further, the above-mentioned homogenate of sputum alone was used as a negative control.

### 4. Collection of microbes

A third filter (pore size: 5 µm, polycarbonate, manufactured by Whatman plc.), a second filter (pore size: 20 µm, nylon, manufactured by Sanplatec Corp.), and a first filter (pore size: 108 µm, nylon, manufactured by Sanplatec Corp.) were charged in order from below in a syringe filter holder and the charged syringe filter holder was connected to a syringe. The resultant was used as a microbe separation device.
An aliquot of each of the prepared suspensions was taken out and passed through the microbe separation device to obtain each filtrate.

### 5. Detection of microbe

Each of the obtained filtrates was centrifuged at room temperature and 3000g for 20 minutes. After a supernatant was removed, the residue was diluted with a dissolving liquid (25 mM of Tris buffer solution (pH 7.0), 0.9% of sodium chloride, 0.1 mM of sodium ethylenediaminetetraacetate, 0. 25% (2000 U/ml) of Bromelain F (manufactured by Amano Enzyme Inc.)) to prepare a S. aureus suspension of 10×10⁶ CFU/ml. The obtained suspension was lysed by the alkali-SDS method (New Biochemical Experiment Course 2 Nucleic acid I Separation and Purification (Tokyo Kagakudojin), Nucleic Acids Res., Vol. 7, p1513 (1979) to prepare a lytic solution.

The obtained lytic solution was detected for nucleic acid of S. aureus with the following method.

### <Preparation of microplate>

A capture probe (nucleic acid probe having the following base sequence) haying a sequence complementary to rRNA of S. aureus was fixed on 96-well microplates each being of strip well type and used for experiments.
Base sequence of the capture probe (SEQ ID NO: 1) 5'-CGTCTTTCACTTTTGAACCATGCGGTTCAAAATATTATCCGG-3'-Amino link

### <Method of preparing each solution>

### (1) Preparation of first hybridization reaction solution

In a first hybridization solution (4XSSC, 0.2% of SDS, 1% of Blocking reagent (manufactured by Roche), 20% of formaldehyde, Salmon sperm DNA (10 µg/ml)) was dissolved an assist probe to make 0.025 pmol/µl of solution to prepare a first hybridization reaction solution. The assist probe is a nucleic acid probe having a sequence complementary to rRNA of S. aureus and the same base sequence as HCP-1 described below, and has the following base sequence.
Base sequence of the assist probe (SEQ ID NO: 2) 5'-CATGTCTCGTGTCTTGCATCCTGCTACAGTGAACACCATCGTTCTCGA CATAGACCAGTCATCTATAAGTGACAGCAAGAC-3'

### (2) Preparation of second hybridization reaction solution

In a second hybridization solution (4XSSC, 0.2% of SDS, 1% of Blocking reagent (manufactured by Roche)) was dissolved HCP-1 and 5'-DIG-labeled HCP-2 to make 1 pmol/µl of solution to prepare a second hybridization reaction solution. The above-mentioned HCP-1 and HCP-2 is a pair of nucleic acid probes used in a PALSAR method. When multiple pairs of probes are reacted, the probes self-assemble to form a polymer of probes (see, for example, Japanese Patent No. 3310662). The base sequences of HCP-1 and HCP-2 are as described below.
Base sequence of HCP-1 (SEQ ID NO: 3) 5'-CATGTCTCGTGTCTTGCATCCTGCTACAGTGAACACCATCGTTCTCGA CATAGACCAGTC-3'
Base sequence of HCP-2 (SEQ ID NO: 4) DIG-5'-GATGCAAGACACGAGACATGGATGGTGTTCACTGTAGCAGGACT GGTCTATGTCGAGAAC-3'

### <Reaction and detection method>

50 µl of the obtained lytic solution and 50 µl each of the first hybridization reaction solutions were dispensed in the wells of the microplate and the microplate was tightly sealed with a plate sealer, followed by reaction for 1 hour (first hybridization). The reaction was performed according to the hybridization method described in WO 2005/106031 under temperature conditions of 20°C on the upper part and 45°C on the lower part of the microplate.
After the reaction, the microplate was washed with a washing liquid (50 mM of Tris, 0.3 M of NaCl, 0.01% of Triton X-100, pH 7.6).

After the washing, the microplate was well-drained of the washing liquid and 100 µl each of the second hybridization reaction solution was dispensed to the wells of the microplate, followed by sealing the microplate with the plate sealer. The reaction was performed under temperature conditions of 20°C on the upper part and 60°C on the lower part of the microplate for 1 hour (second hybridization, PALSAR reaction).

After the microplate was washed, 50 µl of POD-labeled anti-digoxigenin (60 mU/ml) dissolved in 50 mM of Tris (pH 7.6) was dispensed to the wells of the microplate, which was then allowed to react in an incubator at 37°. After the microplate was washed with a washing liquid, 50 µl of a coloring liquid containing 0.2 M of acetate buffer solution (pH 5.0), 0.06% of TMB, and 0.04% of H₂O₂ was added to the wells and the microplate was left in the dark for 15 minutes. Then, absorbance at 655 nm of the sample on each well of the microplate was measured. The results obtained are shown in Fig. 19. On this occasion, the number of microbe was 4.6×10⁵ CFU/well.

### (Example 17)

Experiments were conducted in the same manner as in Example 16 except that the collecting step for collecting the microbe using a microbe separation device was omitted. The results obtained are shown in Fig. 19.

As shown in Fig. 19, in both Examples 16 and 17 using the homogenization treatment of the present invention, the microbe could be detected. In Example 16 in which the microbe was collected using the microbe separation device of the present invention allowed more efficient detection of the microbe from sputum, it was indicated that a reaction inhibitor was removed from sputum by passing the sputum through filters, so the reaction proceeded sufficiently.

### (Example 18)

0. 25% (2000 U/ml) of Bromelain F (manufactured by Amano Enzyme Inc.) was dissolved in a dissolving liquid (25 mM of Tris buffer solution (pH 7.0), 0.9% of sodium chloride, and 0.1 mM of sodium ethylenediaminetetraacetate) to prepare a pretreatment liquid. In a 25-ml container were charged sputum (State P3 purulent sputum), a 10-fold amount based on sputum of the pretreatment liquid, and stainless steel particles (diameter 4.8 mm: 20 grains), and the resultant mixture was allowed to react at room temperature (25°C). On this occasion, the reaction mixture was suspended by application of a vortex (TM-252 TEST TUBE MIXER, manufactured by Asahi Techno Glass Corporation) every 10 minutes. The obtained solution was dissolved for 30 minutes to obtain a homogenate of sputum.

In a syringe filter holder was charged the third filter having a pore size of 5 µm and made of polycarbonate as used in Experimental Example 7 and thereon the second filter having a pore size of 20 µm and made of nylon as used in Experimental Example 10, and further thereon the first filter having a pore size of 108 µm and made of nylon as used in Example 15. This was connected to a syringe and an aliquot of the homogenate of sputum was passed through the filters to obtain homogenate after filtration.

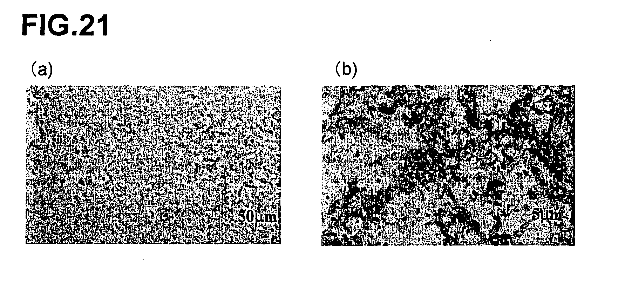

Then, the homogenate before filtration and the homogenate after filtration were each gram-stained using Faber G set S (manufactured by Nissui Pharmaceutical Co., Ltd.) and observed with a microscope. Fig. 20 is a microphotograph of the homogenate before filtration. Fig. 21 is a microphotograph of the homogenate after filtration. In Figs. 20 and 21, (a) relates to the results obtained at a magnification of ×100 and (b) relates to the results obtained at a magnification of ×1000.

The results shown in Fig. 20 indicate that the homogenization method of the present invention completes homogenization of sputum in a short time. In Fig. 20, leucocytes are indicated by an arrow. Further, it was revealed that, as shown in Fig. 21, the homogenate after filtration was free of blood cells and the viscous component of sputum.

## Claims

1. A sampling device for a viscous sample, comprising:
a tubular body (10) with a closed distal end and an open proximal end, having a long straight middle section that is hollow inside, and a flange on an outer periphery of the proximal end;
a gasket (20) inserted in the tubular body so that the gasket is brought into contact with an inner wall of the tubular body and slidable in a longitudinal direction along the tubular body;
a long rod-like plunger (30) having a distal end serving as an engagement convex that engages with the gasket and a proximal end serving as an operation head adapted to slidably operate the gasket when the operation head is operated;
a plate-like nozzle (14) that has substantially the same diameter as that of the straight middle section of the tubular body and is adapted to close the distal end of the tubular body; and
a slit (16) formed in the plate-like nozzle, the slit having a predetermined shape adapted to serve as an aspiration and discharge port for aspirating and discharging the viscous sample.

2. The sampling device for a viscous sample according to claim 1, wherein the slit is positioned near a central part of the plate-like nozzle (14).

3. The sampling device for a viscous sample according to claim 1 or 2, wherein the slit is of a shape having at least one corner (15).

4. The sampling device for a viscous sample according to any one of claims 1 to 3, wherein the shape of the slit is one selected from the group consisting of cross-shape (X-shape), Y-shape, and W-shape.

5. The sampling device for a viscous sample according to any one of claims 1 to 4, wherein the slit has a width of 0.5 mm to 2.0 mm.

6. The sampling device for a viscous sample according to any one of claims 1 to 5, wherein the viscous sample is sputum.

7. An auxiliary jig for the sampling device for a viscous sample according to any one of claims 1 to 6, wherein the auxiliary jig is adapted to make the operation head of the sampling device operable.

8. The auxiliary jig for the sampling device according to claim 7, comprising:
a long tubular external fitted tube member (42) including at a distal end thereof an engagement section engageable with the flange of the tubular body in the sampling device, a proximal end provided with an opening, and a guide groove in a straight middle section of the tube member (42); and
an internal fitted shaft member (50) slidably and coaxially arranged inside the external fitted tube member, and including an engagement section that is engageable with the operation head of the plunger in the sampling device at a distal end thereof, an operation lever along the guide groove, and a press head at a proximal end thereof.

9. The auxiliary jig for the sampling device according to claim 7, comprising:
a long tubular external fitted tube member (62) including on an inner periphery of a distal end thereof a fitting convex fittable with the flange of the tubular body in the sampling device and a proximal end thereof provided with an opening;
an intermediate fitted tube member (70) slidably and coaxially arranged inside the external fitted tube member and including a fitting convex provided on an inner periphery of a distal end thereof adapted to be fittable with the operation head of the plunger in the sampling device and a biasing unit and an engagement unit with respect to the external fitted tube member; and
an internal fitted shaft member (80) slidably and coaxially arranged inside the intermediate fitted tube member and including a distal end serving as an abutting end that abuts the operation head of the plunger in the sampling device and a proximal end serving as a press head.

10. A homogenization agent for sputum, comprising cysteine protease.

11. The homogenization agent according to claim 10, wherein the cysteine protease is at least one member selected from the group consisting of bromelain, papain, chymopapain, and ficin.

12. The homogenization agent according to claim 10 or 11, further comprising sodium chloride and Tris buffer solution.

13. The homogenization agent according to any one of claims 10 to 12, wherein the homogenization agent is used in a pretreatment in a method of detecting a microbe.

14. A homogenization device for homogenizing sputum, comprising at least one particle containing at least one member selected from the group consisting of metals and oxides thereof.

15. The homogenization device according to claim 14, wherein the metals and oxides thereof comprise one of stainless steel, iron and alumina.

16. The homogenization device according to claim 14 or 15, wherein the particle is a spherical particle having a diameter of 1 mm to 10 mm.

17. A homogenization method for sputum, comprising treating sputum with the homogenization agent according to any one of claims 10 to 13.

18. A homogenization method for sputum, comprising stirring a sample containing subject sputum in the presence of at least one particle containing at least one member selected from the group consisting of metals and oxides thereof.

19. The homogenization method for sputum according to claim 18, wherein the metals and oxides thereof comprise one of stainless steel, iron and alumina.

20. The homogenization method for sputum according to claim 18 or 19, wherein the particle is a spherical particle having a diameter of 1 mm to 10 mm.

21. The homogenization method for sputum according to any one of claims 18 to 20, wherein the sample contains a sputum pretreatment agent.

22. The homogenization method for sputum according to claim 21, wherein the sputum pretreatment agent contains a proteolytic enzyme.

23. The homogenization method for sputum according to claim 22, wherein the proteolytic enzyme is at least one member selected from the group consisting of semi-alkaline protease and cysteine protease.

24. The homogenization method for sputum according to claim 21, wherein the sputum pretreatment agent is the homogenization agent according to any one of claims 10 to 13.

25. The homogenization method for sputum according to any one of claims 17 to 24, wherein the method is a pretreatment in detecting a microbe.

26. A method of collecting a microbe from homogenized sputum, comprising:
providing a separation device having at least three types of filters each allowing passage of a subject microbe and made of an organic material; and
passing the homogenized sputum through the separation device to obtain a filtrate containing the subject microbe.

27. The method of collecting a microbe according to claim 26, wherein the homogenized sputum is obtained by the homogenization method for sputum according to any one of claims 17 to 25.

28. The method of collecting a microbe according to claim 26 or 27, wherein the separation device includes a first filter, a second filter, and a third filter arranged in order of passage of the homogenized sputum therethrough, the first filter, the second filter and the third filter having respective pore sizes gradually decreasing in a downstream direction.

29. the method of collecting a microbe according to claim 28, wherein the third filter has a pore size of 0.1 µm to 10 µm.

30. The method of collecting a microbe according to claim 28 or 29, wherein the second filter has a pore size of 5 µm to 30 µm.

31. The method of collecting a microbe according to any one of claims 28 to 30, wherein the first filter has a pore size of 5 µm to 120 µm.

32. The method of collecting a microbe according to any one of claims 26 to 31, wherein the organic material is at least one member selected from the group consisting of polycarbonate, polytetrafluoroethylene, polyamide, cellulose, and polyethylene.

33. The method of collecting a microbe according to any one of claims 26 to 32, wherein the subject microbe is one selected from the group consisting of virus, Rickettsia, bacteria, and fungi.

34. A separation device for separating a microbe, which is the separation device according to any one of claims 26 to 33.

35. A method of detecting a microbe, comprising:
treating a subject sputum by the homogenization method for sputum according to any one of claims 17 to 25; and
detecting a microbe present in the subject sputum.

36. A method of detecting a microbe, comprising:
detecting a microbe from a filtrate obtained by the method of collecting a microbe according to any one of claims 26 to 32.

37. The method of detecting a microbe according to claim 35 or 36, wherein the microbe is one selected from the group consisting of virus, Rickettsia, bacteria, and fungi.
